# EUROPEAN PATENT SPECIFICATION

(11) **EP 3 337 488 B1**
(45) Date of publication and mention of the grant of the patent: **18.12.2024**
(21) Application number: 16836327.3
(22) Date of filing: 19.08.2016
(51) Int. Cl.: A61K 31/7068, A61K 31/7012, A61K 9/00, A61P 31/04, C07H 19/01, C07H 7/02

(54) **TREATMENT AND PREVENTION OF NEISSERIA GONORRHOEAE INFECTION USING CMP-ACTIVATED NONULOSONATE ANALOG COMPOUNDS**
BEHANDLUNG UND PRÄVENTION EINER NEISSERIA-GONORRHOEAE-INFEKTION MITHILFE VON CMP-AKTIVIERTEN NONULOSONAT-ANALOGVERBINDUNGEN
TRAITEMENT ET PRÉVENTION D'INFECTION DE GONOCOQUE À L'AIDE DE COMPOSÉS ANALOGUES DE NONULOSONATE ACTIVÉS PAR CMP

(30) Priority: 19.08.2015 US 201562207246 P
(43) Date of publication of application: 27.06.2018
(73) Proprietor: National Research Council of Canada, Ottawa, K1A 0R6 (CA)
(72) Inventor: SCHOENHOFEN, Ian C., Stittsville, Ontario K2S 2A8 (CA)
(74) Representative: Weickmann & Weickmann PartmbB
(86) International application number: PCT/CA2016/050977
(87) International publication number: WO 2017/027980

(56) References cited:
- CA-A1- 2 843 093
- US-A1- 2012 046 355
- US-A1- 2012 052 532
- US-A1- 2014 356 912
- GESCHE DUFNER ET AL: "Base-and Sugar-Modified Cytidine Monophosphate N-Acetylneuraminic Acid (CMP-Neu5Ac) Analogues - Synthesis and Studies with [alpha](2-6)- Sialyltransferase from Rat Liver", EUR. J. ORG. CHEM, 1 January 2000 (2000-01-01), pages 1467 - 1482, XP055554893, Retrieved from the Internet <URL:https://onlinelibrary.wiley.com/doi/abs/10.1002/%28SICI%291099-0690%28200004%292000%3A8%3C1467%3A%3AAID-EJOC1467%3E3.0.CO%3B2-E> [retrieved on 20190211]
- YU H ET AL: "Chemoenzymatic synthesis of CMP-sialic acid derivatives by a one-pot two-enzyme system: comparison of substrate flexibility of three microbial CMP-sialic acid synthetases", BIOORGANIC & MEDICINAL CHEMI, PERGAMON, GB, vol. 12, no. 24, 15 December 2004 (2004-12-15), pages 6427 - 6435, XP004646484, ISSN: 0968-0896, DOI: 10.1016/J.BMC.2004.09.030
- GULATI ET AL.: "Utilizing CMP-Sialic Acid Analogs to Unravel Neisseria gonorrhoeae Lipooligosaccharide-Mediated Complement Resistance and Design Novel Therapeutics", PLOS PATHOGENS., vol. 13, no. 12, 2 December 2015 (2015-12-02), pages 1 - 26, XP055365367, ISSN: 1553-7366, Retrieved from the Internet <URL:http://journals.plos.orq/plospathoqens/article/asset?id=10.1371/journal.ppat.10 05290.PDF>
- BRAMLEY ET AL.: "A serum-sensitive, sialyltransferase-deficient mutant of Neisseria gonorrhoeae defective in conversion to serum resistance by CMP-NANA or blood cell extracts.", MICROBIAL PATHOGENESIS, vol. 18, no. 3, March 1995 (1995-03-01), pages 187 - 195, XP055365374, ISSN: 0882-4010
- LEWIS ET AL.: "a-2,3-sialyltransferase expression level impacts the kinetics of lipooligosaccharide sialylation, complement resistance, and the ability of Neisseria gonorrhoeae to colonize the murine genital tract.", MBIO., vol. 6, no. 1, 3 February 2015 (2015-02-03), pages 1 - 11, XP055363426, ISSN: 2150-7511

## Description

### FIELD OF THE INVENTION

The present invention relates to medical conditions involving *Neisseria gonorrhoeae.* More specifically, the present invention relates to cytidine 5'-monophospho-nonulosonate (CMP-NulO) analog compounds for use in treating and preventing *Neisseria gonorrhoeae* infection.

### BACKGROUND OF THE INVENTION

Sialic acids are a family of 9 carbon sugars (belonging to a larger family of nonoses, or nonulosonates) expressed in the tissues of every vertebrate and several "higher-order" invertebrates [1]. Sialic acids serve a wide variety of biological roles, including modulating several aspects of immune function [2]. For example, cell surface-associated sialic acid inhibits complement activation. As an example of immune regulation, sheep erythrocytes are resistant to lysis by the alternative pathway because surface sialic acids increase the affinity of factor H (fH; inhibitor of the alternative pathway) [3]. Neuraminidase treatment of sheep erythrocytes then reduces the affinity of fH, which permits complement activation and promotes hemolysis. Recent work showed that fH C-terminal domains 19 and 20 bound simultaneously to C3b (complement factor that binds microbial cell surfaces) and glycosaminoglycans (including sialic acids), respectively, on host cells, which served to inhibit the alternative pathway [4]. Loss of sialic acids decreased fH binding and enhanced activation of the alternative pathway. Typically, fH binds vertebrate cell surfaces via sialic acids to allow preferential protection of host cells (i.e. reduce complement-mediated damage).

Many microbes express sialic acids, as well as other unique microbial nonulosonates (i.e. legionaminic (Leg) and pseudaminic (Pse) acid), on their surfaces that contribute to pathogenesis in several ways including subversion of complement activation, promoting biofilm formation and facilitating colonization [5]. Some pathogens such as *Neisseria gonorrhoeae, Haemophilus influenzae, Histophilus somni (Haemophilus somnus*) and group A *N. meningitidis* lack the ability to synthesize sialic or nonulosonic acids, but scavenge these molecules (such as Neu5Ac or Neu5Gc, or the CMP-activated form CMP-Neu5Ac) from the host. Other pathogens, for example, *Escherichia coli* K1, *Streptococcus agalactiae,* groups B, C, W, and Y *N. meningitidis, Campylobacter jejuni* and certain *Leptospira,* can synthesize nonulosonic acids such as Neu5Ac, Leg5Ac7Ac or Pse5Ac7Ac de novo. Sialylation of gonococcal lacto-*N-*neotetraose (LNnT) lipooligosaccharide (LOS) enhances resistance of *N*. *gonorrhoeae* to complement-dependent killing by decreasing binding of IgG against select bacterial targets such as the porin B (PorB) protein [6], which attenuates the classical pathway. LNnT LOS sialylation also enhances fH binding, which results in inhibition of the alternative pathway [7].

*N. gonorrhoeae* has become resistant to almost every conventional antibiotic. Over the past 3 years, resistance to ceftriaxone has ushered in an era of potentially untreatable gonorrhea. There is an urgent need for novel therapeutics and vaccines against this disease. LOS sialylation is an important aspect of gonococcal pathogenesis and isogenic mutants that lack the ability to sialylate their LOS are at a disadvantage *in vivo* compared to their wild-type counterparts [8]. Disabling the ability of gonococci to sialylate their LOS represents a novel prophylactic or treatment strategy.

U.S. Patent Application Serial No. 14/627,396 discloses cytidine 5'-monophospho-nonulosonate (CMP-NulO) analog compounds for treating or preventing *Neisseria gonorrhoeae* infection in a subject.

Also, the inventor is aware of these other documents [36-46].

US 2014/356912 A1 relates to the cell-based production of bacterial nonulosonates and their biosynthetic precursors. Specifically, recombinant cells for the production of pseudaminic acid, legionaminic acid, UDP-2,4-diacetamido-2,4,6-trideoxy-β-L-altropyranose, and UDP-2,4-diacetamido-2,4,6-trideoxy-α-D-glucopyranose are provided. Methods for producing the sugars are also provided.

Bramley et al. (Microbial Pathogenesis 1995, vol.18, pp.187-195) describe a serum-sensitive, sialyltransferase-deficient mutant of *Neisseria gonorrhoeae.* The mutant, JT1, is deficient in the sialyltransferase that is essential for both LPS sialylation and conversion of serum-sensitive gonococci to serum resistance by either CMP-NANA or blood cell extracts. No evidence was obtained for an LPS sialylation pathway by blood cell extracts that is independent of CMP-NANA. If

Lewis et al. (MBIO, 2015, vol. 6, e02465-14) describe the significance of differential Ist expression levels and determined that the level of LOS sialylation is critical to the ability of N. gonorrhoeae to combat the immune system and survive in an animal model. Level of LOS sialylation was determined by incorporation of tritium-labeled cytidine monophospho-N-acetylneuraminic acid (CMP-NANA).

There is a need for CMP-NulO analog compounds that provide a more efficient treatment or prevention. Also, there is a need for CMP-NulO analog compounds that present low toxicity effects in a subject.

### SUMMARY OF THE INVENTION

The invention is drawn to cytidine 5'-monophospho-nonulosonate (CMP-NulO) analog compounds for use in of treating or preventing *Neisseria gonorrhoeae* infection in a subject. More specifically, the compounds of the invention relate to CMP-3-deoxy-D-*glycero*-D-*galacto*-nonulosonic acid (**CMP-KDN**). Since 3-deoxy-D-*glycero*-D-*galacto-*nonulosonic acid (KDN, also called 3-deoxy-D-*glycero*-D-*galacto*-2-nonulosonic acid or 2-keto-3-deoxy-D-*glycero*-D-*galacto*-nononic acid) is a sugar found in humans at low levels, it is anticipated that any toxic effects associated to the use of the compounds of the invention will be low.

The invention thus provides the following according to aspects thereof:
(1). In one aspect, the invention provides a compound of general formula **IIA** below or a pharmaceutical composition comprising said compound, or a pharmaceutically acceptable salt thereof, or a solvate or hydrate thereof, or a stereoisomer thereof, for use in the treatment or prevention of a *Neisseria gonorrhoeae* infection in a subject 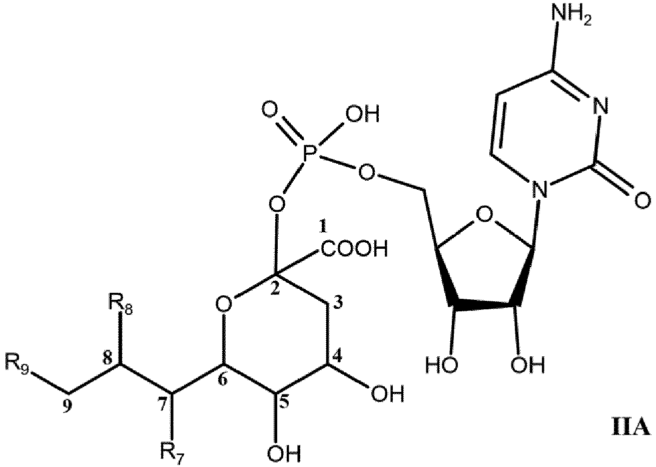 wherein:
   R₇ to R₉ are each independently selected from the group consisting of: H; OR wherein R is H or a C₁ to C₆ linear, branched, saturated or unsaturated alkyl or cycloalkyl; XCYR wherein X and Y are each independently O or S and R is a C₁ to C₆ linear, branched, saturated or unsaturated alkyl or cycloalkyl or R is a substituted or unsubstituted phenyl or alkyl phenyl; NR'R" wherein R' and R" are each independently H, a C₁ to C₆ linear, branched, saturated or unsaturated alkyl or cycloalkyl, or R' and R" together with N form a 5- or 6-member ring, optionally the ring is substituted with a C₁ to C₃ alkyl; NH-acetyl; NH-thio-acetyl; NH-azido-acetyl; NH-(D-alanyl); NH-(N-acetyl-D-alanyl); N₃; O-Sia; O-Glc; benzamido [NHCOPh]; NH-Gly; NH-Succ; hexanoylamido [NHCO(CH₂)₄CH₃]; O-lactyl; O-phosphate; O-sulfate; and a halogen atom which is F, Cl, Br or I.
(2). In one embodiment of the invention:
   R₇ is OH, NH₂, O-acetyl, O-methyl, NH-acetyl, NH-azido-acetyl, NH-(D-alanyl), NH-(N-acetyl-D-alanyl), F, H, or N₃;
   R₈ is OH, NH₂, N₃, O-acetyl, O-methyl, O-sulfate, O-Sia, or O-Glc; and
   R₉ is OH, O-acetyl, N₃, NH₂, NH-acetyl, NH-thio-acetyl, benzamido [NHCOPh], NH-Gly, NH-Succ, hexanoylamido [NHCO(CH₂)₄CH₃], O-methyl, O-lactyl, O-phosphate, O-sulfate, O-Sia, F or H.
(3). In an embodiment of the invention, the compound is compound V below 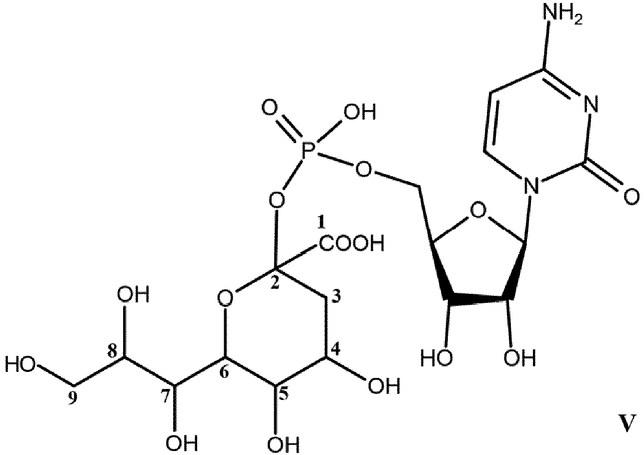
(4). In an embodiment of the invention, the compound is cytidine 5'-monophospho-3-deoxy-D-*glycero*-D-*galacto*-nonulosonic acid (**CMP-KDN**) below 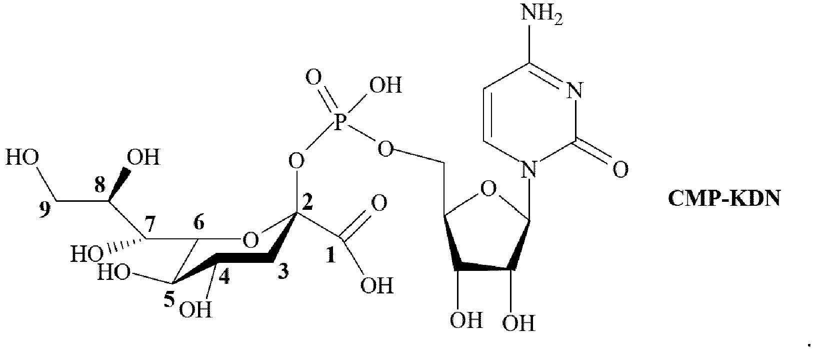
(5). In an embodiment of the invention, the compound is cytidine 5'-monophospho-3,7-dideoxy-7-azido-D-*glycero*-D-*galacto*-nonulosonic acid (**CMP-KDN7N₃**) below 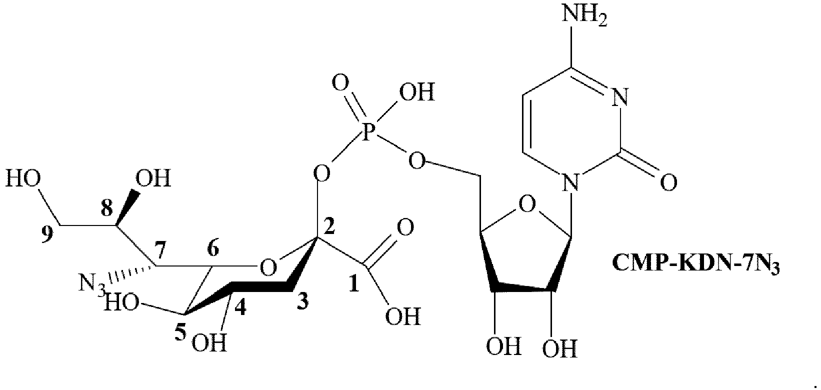
(6). In an embodiment of the invention, the compound is:
   cytidine 5'-monophospho-3-deoxy-9-O-acetyl-D-*glycero*-D-*galacto*-nonulosonic acid (CMP-KDN9OAc) (R₇ = OH, R₈ = OH, R₉= O-acetyl);
   cytidine 5'-monophospho-3-deoxy-8-O-acetyl-D-*glycero*-D-*galacto*-nonulosonic acid (CMP-KDN8OAc) (R₇ = OH, R₈ = O-acetyl, R₉= OH);
   cytidine 5'-monophospho-3-deoxy-7-O-acetyl-D-*glycero*-D-*galacto*-nonulosonic acid (CMP-KDN7OAc) (R₇ = O-acetyl, R₈ = OH, R₉= OH);
   cytidine 5'-monophospho-3-deoxy-8,9-di-O-acetyl-D-*glycero*-D-*galacto*-nonulosonic acid (CMP-KDN8,9diOAc) (R₇ = OH, R₈ = O-acetyl, R₉= O-acetyl);
   cytidine 5'-monophospho-3-deoxy-9-O-methyl-D-*glycero*-D-*galacto*-nonulosonic acid (CMP-KDN9OMe) (R₇ = OH, R₈ = OH, R₉ = O-methyl);
   cytidine 5'-monophospho-3-deoxy-8-O-methyl-D-*glycero*-D-*galacto*-nonulosonic acid (CMP-KDN8OMe) (R₇ = OH, R₈ = O-methyl, R₉= OH);
   cytidine 5'-monophospho-3-deoxy-7-O-methyl-D-*glycero*-D-*galacto*-nonulosonic acid (CMP-KDN7OMe) (R₇ = O-methyl, R₈ = OH, R₉= OH);
   cytidine 5'-monophospho-3-deoxy-8,9-di-O-methyl-D-*glycero*-D-*galacto*-nonulosonic acid (CMP-KDN8,9diOMe) (R₇ = OH, R₈ = O-methyl, R₉= O-methyl);
   cytidine 5'-monophospho-3,9-dideoxy-D-*glycero*-D-*galacto*-nonulosonic acid (CMP-9-deoxy-KDN) (R₇ = OH, R₈ = OH, R₉= H);
   cytidine 5'-monophospho-3,7-dideoxy-D-*glycero*-D-*galacto*-nonulosonic acid (CMP-7-deoxy-KDN) (R₇ = H, R₈ = OH, R₉ = OH);
   cytidine 5'-monophospho-3,9-dideoxy-9-azido-D-*glycero*-D-*galacto*-nonulosonic acid (CMP-KDN9Az) (R₇ = OH, R₈ = OH, R₉= N₃);
   cytidine 5'-monophospho-3,9-dideoxy-9-fluoro-D-*glycero*-D-*galacto*-nonulosonic acid (CMP-KDN9F) (R₇ = OH, R₈ = OH, R₉= F); or
   cytidine 5'-monophospho-3,7-dideoxy-7-fluoro-D-*glycero*-D-*galacto*-nonulosonic acid (CMP-KDN7F) (R₇ = F, R₈ = OH, R₉= OH).
(7). In an embodiment of the invention the pharmaceutical composition comprises the compound and a pharmaceutically acceptable carrier.
(8). In an embodiment of the invention the pharmaceutical composition comprises the compound and another therapeutic compound.
(9). In an embodiment, the another therapeutic compound is selected from the group consisting of: other compounds used in the treatment or prevention of *Neisseria gonorrhoeae* infection, compounds used in the treatment or prevention of sexually transmitted diseases including *Chlamydia trachomatis* infection and HIV, and antibacterial peptides.
(10). In an embodiment of the invention, the subject is a mammal.
(11). In an embodiment of the invention the subject is a human.
(12). In one aspect, the invention provides a device coated or filled with the compound or the pharmaceutical composition for use as defined herein.

Other objects, advantages and features of the present invention will become more apparent upon reading of the following non-restrictive description of specific embodiments thereof, given by way of example only with reference to the accompanying drawings.

### BRIEF DESCRIPTION OF THE DRAWINGS

Example embodiments are illustrated by way of example in the figures of the accompanying drawings, in which:

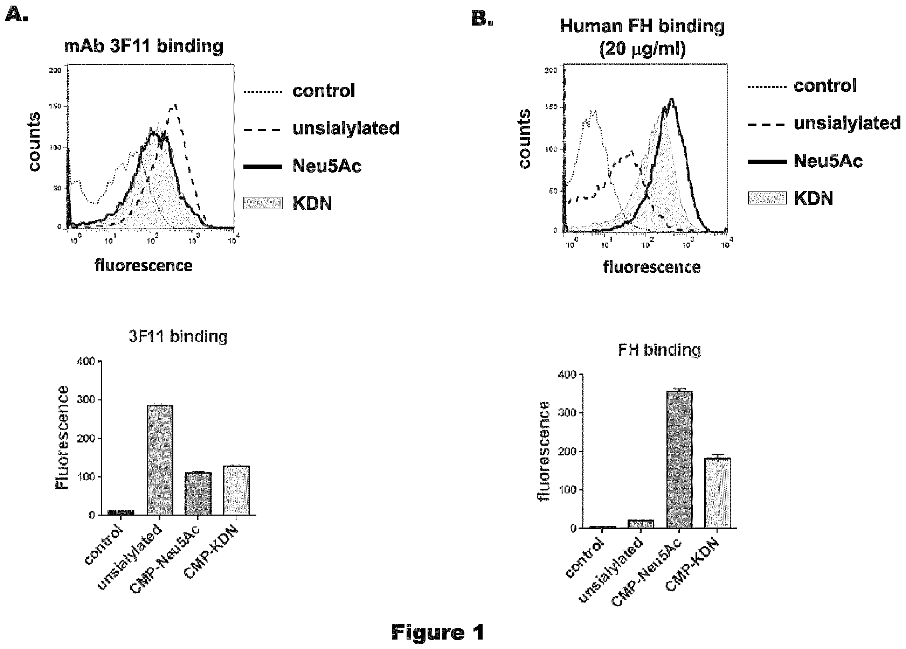

**Figure 1****: CMP-KDN** serves as a substrate for gonococcal LOS and incorporation of KDN on gonococcal lacto-N-neotetraose LOS enhances factor H (FH) binding. **A.** Incorporation of KDN by gonococci. Gonococcal strain F62 ΔIgtD (expresses lacto-N-neotetraose (LNnT) from Hepl) was grown in media containing **CMP-KDN** (20 µg/mL) and binding of mAb 3F11 was measured by flow cytometry. mAb 3F11 binds only to sialylated LNnT; substitution of the terminal Gal of LNnT, for example with a NulO, will decrease mAb 3F11 binding. Approximately 5 x 10⁷ bacteria were incubated with mAb 3F11 tissue culture supernatants for 15 minutes at 37°C. Binding of mAb 3F11 was disclosed with anti-mouse IgM FITC (Sigma; dilution of 1:100). Bacteria grown in CMP-Neu5Ac or in media alone were used as positive and negative controls for sialylation, respectively. A representative histogram is shown in the upper panel. The bar graph shows the average of the median fluorescence from two independent observations. **B.** Factor H (FH) binding to gonococci grown in **CMP-KDN.** Approximately 5 x 10⁷ bacteria grown in media alone (no sialic acid), media plus CMP-Neu5Ac (20 µg/mL; positive control for FH binding) or in media containing **CMP-KDN** (20 µg/mL) were incubated with purified human FH (20 µg/mL) in HBSS⁺⁺ for 15 minutes at 37°C and bound FH was detected with affinity isolated goat anti-human FH followed by anti-goat IgG FITC (Sigma; 1:100 dilution). A representative histogram is shown in the upper panel. The bar graph shows the average of the median fluorescence from two independent observations.

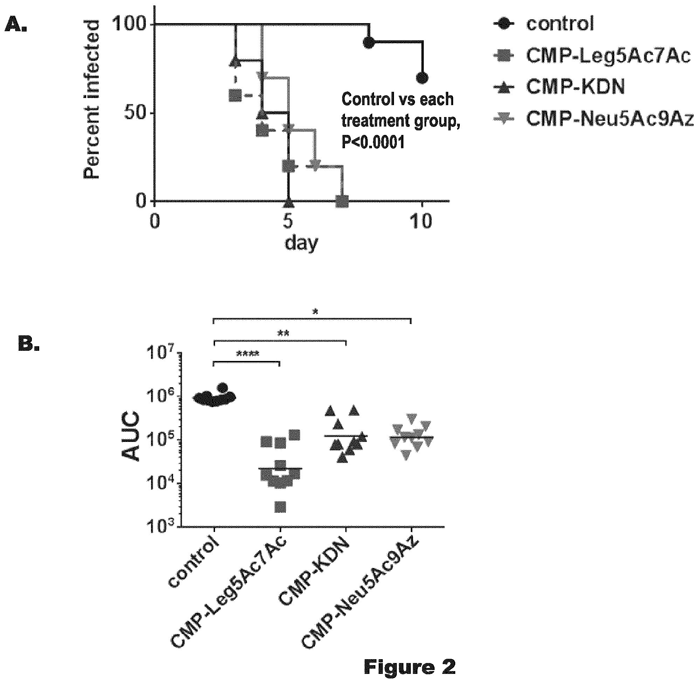

**Figure 2****:** Female BALB/c mice 5-6 weeks of age (Jackson Laboratories) in the diestrus phase of the estrous cycle were started on treatment (that day) with 0.5 mg of Premarin (Pfizer) given subcutaneously on each of three days; -2, 0 and +2 days (before, the day of and after inoculation) to prolong the estrus phase of the cycle and promote susceptibility to *N. gonorrhoeae* infection. Antibiotics (vancomycin, colistin, neomycin, trimethoprim and streptomycin) that were ineffective against *N. gonorrhoeae* were also used to reduce competitive microflora. Mice (n=40) were then infected with 10⁶ CFU of strain H041. Three groups of mice (n=10 / group) were treated with 10 µg intravaginally daily (first dose was administered 30 minutes before the introduction of bacteria) with one of the following CMP-NulOs in normal saline: CMP-Leg5Ac7Ac, CMP-Neu5Ac9Az or **CMP-KDN.** A fourth group (n=10) was given saline (vehicle control). Vaginal swabs were obtained daily from each animal, serially diluted and plated on chocolate agar containing vancomycin, colistin, neomycin, trimethoprim and streptomycin (VCNTS) to quantify bacterial loads. **A.** Median time to clearance was estimated using Kaplan-Meier survival curves; the times to clearance were compared between groups using a log-rank test. P<0.0001 for the control versus each of the treatment groups. Time to clearance between each of the treatment groups was similar (P > 0.05). **B.** The mean area under the curve (log₁₀CFU vs. time) was computed for each mouse to estimate the bacterial burden over time (cumulative infection); the means under the curves were compared between groups using the Kruskal-Wallis nonparametric rank sum test because distributions were skewed or kurtotic. Groups were compared using Dunn's multiple comparison test. ****, P<0.0001; **, P<0.01; *, P<0.05. The differences among the groups that received CMP-NulOs were not significant.

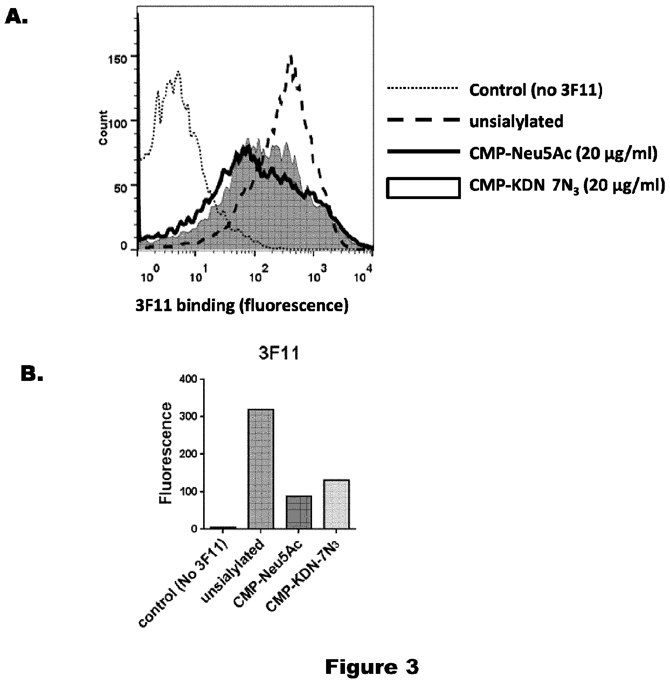

**Figure 3****: CMP-KDN7N₃** serves as a substrate for gonococcal Lst resulting in incorporation of KDN7N₃ on gonococcal lacto-N-neotetraose (LNnT) LOS - Incorporation of KDN7N₃ by gonococci. Gonococcal strain F62ΔlgtD (expresses lacto-N-neotetraose (LNnT) from Hepl) was grown in media containing **CMP-KDN7N₃** (20 µg/mL) and binding of mAb 3F11 was measured by flow cytometry. mAb 3F11 binds only to sialylated LNnT; substitution of the terminal Gal of LNnT, for example with a NulO, will decrease mAb 3F11 binding. Approximately 5 x 10⁷ bacteria were incubated with mAb 3F11 tissue culture supernatants for 15 minutes at 37°C. Binding of mAb 3F11 was disclosed with anti-mouse IgM FITC (Sigma; dilution of 1:100). Bacteria grown in CMP-Neu5Ac or in media alone were used as positive and negative controls for sialylation, respectively. A representative histogram is shown in the upper panel (**A**), and a bar graph below shows the median fluorescence for each condition (**B**).

Other features of the present embodiments will be apparent from the accompanying drawings and from the detailed description that follows.

### DESCRIPTION OF ILLUSTRATIVE EMBODIMENTS

In order to provide a clear and consistent understanding of the terms used in the present specification, a number of definitions are provided below. Moreover, unless defined otherwise, all technical and scientific terms as used herein have the same meaning as commonly understood to one of ordinary skill in the art to which this disclosure pertains.

The use of the word "a" or "an" when used in conjunction with the term "comprising" in the claims and/or the description may mean "one", but it is also consistent with the meaning of "one or more", "at least one", and "one or more than one". Similarly, the word "another" may mean at least a second or more.

As used herein, the words "comprising" (and any form of comprising, such as "comprise" and "comprises"), "having" (and any form of having, such as "have" and "has"), "including" (and any form of including, such as "include" and "includes") or "containing" (and any form of containing, such as "contain" and "contains"), are inclusive or open-ended and do not exclude additional, unrecited elements or process steps.

As used herein, the term "effective amount" is an amount of the CMP-nonulosonate analog compound that is sufficient to treat a *N. gonorrhoeae* infection, that is, to accomplish at least one of the following: reduce virulence of *N. gonorrhoeae,* reduce the rate of transmission of *N. gonorrhoeae,* and reduce the severity of one or more symptoms associated with *N. gonorrhoeae* infection, for example, burning sensation during urination, painful or swollen testicles and increased vaginal discharge.

As used herein, the term "subject" is understood as being any mammal including a human being treated with a compound of the invention.

As used herein, the term "salt" is understood as being acidic and/or basic salts formed with inorganic and/or organic acids or bases. Zwitterions (internal or inner salts) are understood as being included within the term "salt" as used herein, as are quaternary ammonium salts such as alkylammonium salts. Nontoxic, pharmaceutically acceptable salts are preferred, although other salts may be useful, as for example in isolation or purification steps.

Examples of acid addition salts include but are not limited to acetate, adipate, alginate, aspartate, benzoate, benzenesulfonate, bisulfate, butyrate, citrate, camphorate, camphorsulfonate, cyclopentanepropionate, digluconate, dodecylsulfate, ethanesulfonate, fumarate, glucoheptanoate, glycerophosphate, hemisulfate, heptanoate, hexanoate, hydrochloride, hydrobromide, hydroiodide, phosphoric, 2-hydroxyethanesulfonate, lactate, maleate, mandelate, methanesulfonate, 2-naphthalenesulfonate, nicotinate, oxalate, pectinate, persulfate, 3-phenylpropionate, picrate, pivalate, propionate, succinate, tartrate, thiocyanate, tosylate, and undecanoate.

Salts may also be made from inorganic acids such as hydrochloric, hydrobromic, sulfuric, sulfamic, phosphoric and nitric acids.

Examples of base addition salts include but are not limited to alkali metal salts and alkaline earth metal salts. Non limiting examples of alkali metal salts include lithium, sodium and potassium salts. Non-limiting examples of alkaline earth metal salts include magnesium and calcium salts.

As used herein the term "therapeutically effective amount" of a compound means an amount sufficient to cure, alleviate or partially arrest the clinical manifestations of a given disease and its complications in a therapeutic intervention comprising the administration of said compound. An amount adequate to accomplish this is defined as "a therapeutically effective amount". Effective amounts for each purpose will depend on the severity of the disease or injury as well as the weight and general state of the subject.

As used herein the terms "treatment" and "treating" mean the management and care of a subject for the purpose of combating a condition, such as a disease or disorder. The term is intended to include the full spectrum of treatments for a given condition from which the patient is suffering, such administration of the active compounds to alleviate the symptoms or complications, to delay the progression of the condition, and/or to cure or eliminate the condition. The subject to be treated is preferably a mammal, in particular a human being.

The present disclosure is drawn to cytidine 5'-monophospho-nonulosonate (CMP-NulO) analog compounds for use in a method of treating or preventing *Neisseria gonorrhoeae* infection in a subject. More specifically, the compounds of the invention relate to CMP-3-deoxy-D-*glycero*-D-*galacto*-nonulosonic acid (**CMP-KDN**). Since 3-deoxy-D-glycero-D-galacto-nonulosonic acid (KDN, also called 3-deoxy-D-*glycero*-D-*galacto*-2-nonulosonic acid or 2-keto-3-deoxy-D-*glycero*-D-*galacto*-nononic acid) is a sugar found in humans at low levels, it is anticipated that any toxic effects associated to the use of the compounds of the invention will be low.

Indeed, 3-deoxy-D-*glycero*-D-*galacto*-nonulosonic acid (KDN) is a sialic acid or nonulosonate (NulO) that is ubiquitously expressed in vertebrates during normal development and tumorigenesis. In KDN, the N-acetyl group at C5 of N-acetyl-neuraminic acid is replaced by a hydroxyl group, and again is found in vertebrate glycoconjugates and bacterial polysaccharides, where it was first identified in rainbow trout egg polysialoglycoprotein in 1986 [9,10]. Its expression is thought to involve i) mannose-6-phosphate + phosphoenolpyruvate (PEP) -> KDN-9-phosphate (KDN-9-P) + Pi; ii) KDN-9-P -> KDN + Pi; iii) KDN + CTP -> CMP-KDN + PPi; and iv) CMP-KDN + R-OH -> R-O-KDN + CMP (R, acceptor glycan) [9,10]. In summary, KDN occurs widely among vertebrates and bacteria, is found in almost all types of glycoconjugates, can be linked to almost all glycan structures in place of Neu5Ac, and its biosynthesis involves mannose, CMP-activation of KDN and transfer to acceptor sugar residues [10].

Using crude enzyme preparations, it has been shown that mammalian CMP-sialic acid synthetases (enzymes responsible for step iii) above) have very low activity/ability to synthesize **CMP-KDN** from KDN and CTP, relative to enzymes from rainbow trout [11,12]. In humans, the Neu5Ac-9-phosphate synthase (step i) above) can catalyze the synthesis of both Neu5Ac-9-phosphate and KDN-9-phosphate from aldol condensation of PEP with substrates ManNAc-6-phosphate or Man-6-phosphate, respectively [13-15]. In addition, the human CMP-sialic acid synthetase can CMP-activate KDN [14]. Importantly, although a minor component, KDN has been reported to be present in human tissues [16-19] and therefore is likely to be poorly immunogenic. Moreover, KDN in the context of glycoconjugates has been demonstrated to be sialidase resistant [20,21]. Due to the inherent presence of KDN in human tissues, its sialidase resistance (i.e., stability) and its relative ease of chemical synthesis [22,23], **CMP-KDN** is an attractive therapeutic agent for humans.

The compounds of the invention are of general formula **IIA, V, VI, VII** as outlined below. 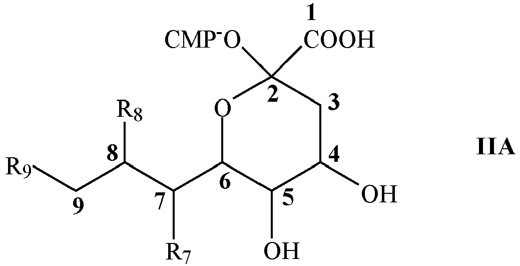 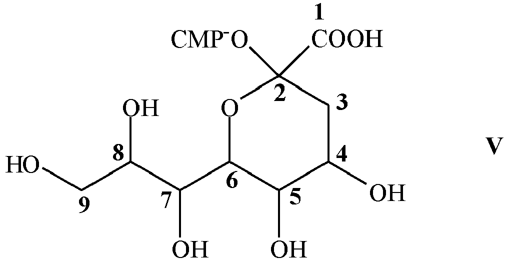 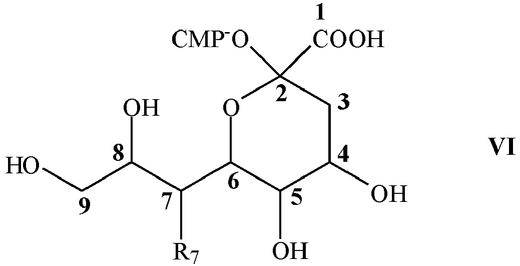 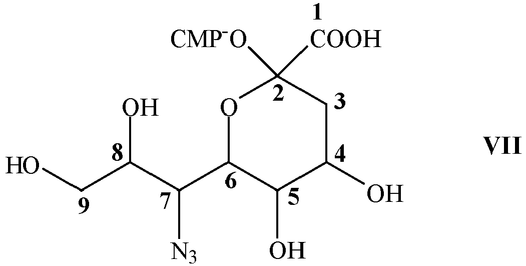 wherein:
R₇ to R₉ are each independently selected from the group consisting of: H; OR wherein R is H or a C₁ to C₆ linear, branched, saturated or unsaturated alkyl or cycloalkyl; OR'R" wherein R' and R" are each independently H or a C₁ to C₆ linear, branched, saturated or unsaturated alkyl or cycloalkyl; XCYR wherein X and Y are each independently O or S and R is a C₁ to C₆ linear, branched, saturated or unsaturated alkyl or cycloalkyl or R is a substituted or unsubstituted phenyl or alkyl phenyl; NR'R" wherein R' and R" are each independently H, a C₁ to C₆ linear, branched, saturated or unsaturated alkyl or cycloalkyl, or R' and R" together with N form a 5- or 6-member ring, optionally the ring is substituted with a C₁ to C₃ alkyl; NH-acetyl; NH-thio-acetyl; NH-azido-acetyl; NH-(D-alanyl); NH-(N-acetyl-D-alanyl); N₃; O-Sia; O-Glc; benzamido [NHCOPh]; NH-Gly; NH-Succ; hexanoylamido [NHCO(CH₂)₄CH₃]; O-lactyl; O-phosphate; O-sulfate; and a halogen atom which is F, Cl, Br or I.

Also, compounds of the invention include compound **CMP-KDN** and compound **CMP-KDN7N₃** outlined below.

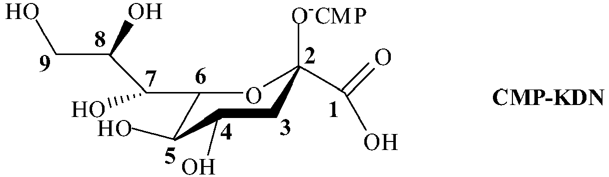

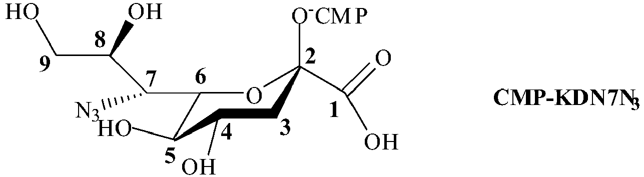

Moreover, compounds of the invention include the following:
In some embodiments, the formulation may include or may further comprise enzymatic inhibitors, pH modulating compounds, buffers, salt formation, solubilizers, excipients, emulsifiers, surfactants and/or antioxidants or the like. Such pharmaceutical compositions are also envisioned and are within the scope of the invention. For example, the formulation may include a sialyltransferase, for example, Lst or another suitable sialyltransferase in the formulation.

In other embodiments, the formulation may be a sustained release formulation. The term "sustained release" as used herein refers to the release of a drug or compound at a predetermined rate in order to maintain a specific concentration for a specific period of time. Sustained release formulations are well known in the art and may comprise for example a hydrogel, liposomes or a polymer.

In some embodiments, the formulation may include or may further comprise enzymatic inhibitors, pH modulating compounds, buffers, salt formation, solubilizers, excipients, emulsifiers, surfactants and/or antioxidants or the like. Such pharmaceutical compositions are also envisioned and are within the scope of the invention. For example, the formulation may include a sialyltransferase, for example, Lst or another suitable sialyltransferase in the formulation.

Suitable products will be readily apparent to one of skill in the art. For example, one or more of the CMP-nonulosonate sugars of the invention may be formulated for intravenous or topical administration, as discussed herein.

For oral administration, the CMP-nonulosonate analog compounds may be formulated in a tablet, coated tablet, capsule or other similar form known in the art for oral administration of medicaments.

For topical administration, the CMP-nonulosonate analog compounds may be formulated in a spray, cream, lotion, ointment or similar product, as well as a device similar to that used for yeast infections (i.e., including tablet or the like). This device could be used for treatment and prophylaxis. In other embodiments, the CMP-nonulosonate analog compounds may be formulated for release from a prophylactic device. Examples of suitable prophylactic devices include but are by no means limited to condoms, cervical caps, contraceptive diaphragms, vaginal rings, devices used for yeast infections and the like.

As will be appreciated by one of skill in the art, in some embodiments of the invention, the formulation comprises an effective amount of one or more of the cytidine 5'-monophospho-nonulosonate analog compounds and is used as a treatment for a subject who has or is suspected of having or is at risk of a *N. gonorrhoeae* infection. For example, the cytidine 5'-monophospho-nonulosonate sugars may be incorporated into a formulation as disclosed herein and formulated as a medicament for treatment of a *N. gonorrhoeae* infection. For example, the medicament comprising the cytidine 5'-monophospho-nonulosonate sugars may be formulated for oral, intravenous administration or for topical administration. As discussed herein, the medicament may also be formulated for sustained release.

In other embodiments of the invention, there is provided a prophylactic device coated or filled with an effective amount of one or more of the cytidine 5'-monophospho-nonulosonate analog compounds defined above for treating an individual who has or is suspected of having or is at risk of an *N. gonorrhoeae* infection. In some embodiments, the cytidine 5'-monophospho-nonulosonate analog compound is formulated for sustained release, as discussed above.

As will be appreciated by one of skill in the art, a subject who is "at risk" of a *N. gonorrhoeae* infection is a subject who may have sexual contact with another subject who may be infected by *N. gonorrhoeae.*

### Description of a preferred embodiment

### Synthesis of CMP-3-deoxy-D-glycero-D-galacto-nonulosonic acid or CMP-deaminated neuraminic acid (CMP-KDN)

KDN (3-deoxy-D-*glycero*-D-*galacto*-nonulosonic acid) was enzymatically prepared using a *Pasteurella multocida* aldolase [24]. Typically, reactions contained 100 mM Tris pH 7.5, 20 mM mannose, 100 mM sodium pyruvate, and approximately 0.15 mg/mL aldolase. Reactions were incubated at 37°C with gentle shaking for 24-48 hours, and finally enzyme was removed by centrifugal ultrafiltration. Next, CMP-activation of synthesized KDN was achieved enzymatically using a CMP-sialic acid synthetase from *Campylobacter jejuni* [25]. Here, reactions typically contained 50 mM Tris pH 8.5, 50 mM MgCl₂, 5 mM CTP, approximately 5 mM KDN, 4 units pyrophosphatase per mmole of CTP and approximately 0.1 mg/mL of CMP-sialic acid synthetase. Reactions were incubated at 37°C for 2 hours, and finally enzyme was removed by centrifugal ultrafiltration. The filtered CMP-KDN was then purified using a Q sepharose fast flow (GE Healthcare) column equilibrated in 1 mM NaCl. Before sample application, the CMP-KDN preparation was diluted approximately 40 times in 1 mM NaCl. After sample application, the resin was washed with 2 CV of 1 mM NaCl and purified **CMP-KDN** was obtained with a 0.8 CV 100 mM NaCl step elution. This CMP-KDN preparation was further desalted using diafiltration, where the sample was transferred to a diafiltration cell (Diaflo ultrafiltration membranes, YCO5 76 mm), and filtered using 3 times the volume of 1 mM NaCl at a flow rate of 32 mL/h. After 24 hours, the retentate was isolated containing approximately 96% of the original CMP-KDN. Quantification of **CMP-KDN** preparations were determined using the molar extinction coefficient of CMP (ε260=7,400). Purified and desalted sample aliquots were then freeze dried.

### Synthesis of CMP-3,7-dideoxy-7-azido-D-glycero-D-galacto-nonulosonic acid (CMP-KDN7N₃)

KDN7N₃ (3,7-dideoxy-7-azido-D-glycero-D-galacto-nonulosonic acid) was enzymatically prepared using a *Pasteurella multocida* aldolase [24]. Typically, reactions contained 128 mM Tris pH 8.8, 17.5 mM 4-azido-4-deoxy-D-mannopyranose (Sussex Research Laboratories Inc.), 128 mM sodium pyruvate, and sufficient quantities of aldolase. Reactions were incubated at 37°C for approximately 24 hours, and finally enzyme was removed by centrifugal ultrafiltration. Next, CMP-activation of synthesized KDN7N₃ was achieved enzymatically using a CMP-sialic acid synthetase from *Campylobacter jejuni* [25]. Here, reactions typically contained 50 mM Tris pH 9, 50 mM MgCl₂, 5 mM CTP, approximately 5 mM KDN7N₃, 4 units pyrophosphatase per mmole of CTP and approximately 0.68 mg/mL of CMP-sialic acid synthetase. Reactions were incubated at 37°C for 2 hours, and finally enzyme was removed by centrifugal ultrafiltration. Filtered **CMP-KDN7N₃** samples were then lyophilized and desalted/purified using a Superdex Peptide 10/300 GL (GE Healthcare) column with 10 mM ammonium bicarbonate. To achieve additional purity, elution fractions containing **CMP-KDN7N₃** were subjected to anion-exchange chromatography (Mono Q 4.6/100 PE, GE Healthcare) using an ammonium bicarbonate gradient. Quantification of **CMP-KDN7N₃** preparations were determined using the molar extinction coefficient of CMP (ε260=7,400). Prior to lyophilization, NaCl was added to **CMP-KDN7N₃** preparations in a molar ratio of 2:1 (salt: NulO).

For structural characterization of **CMP-KDN** and **CMP-KDN7N₃,** purified material was exchanged into 100% D₂O. Structural analysis was performed using either a Varian Inova 500 MHz (¹H) spectrometer with a Varian Z-gradient 3-mm probe or a Varian 600 MHz (¹H) spectrometer with a Varian 5 mm Z-gradient probe. All spectra were referenced to an internal acetone standard (δ_{H} 2.225 ppm and δ_{C} 31.07 ppm). Results are shown in **Table 2 (CMP-KDN)** and **Table 3 (CMP-KDN7N₃)** below verifying the production of each compound.

**CMP-KDN** and **CMP-KDN7N₃** prepared compounds were also characterized using mass spectrometry (MS) or CE-MS analysis. For CE-MS, mass spectra were acquired using an API3000 mass spectrometer (Applied Biosystems/Sciex, Concord, ON, Canada). CE was performed using a Prince CE system (Prince Technologies, Netherlands). CE separation was obtained on a 90 cm length of bare fused-silica capillary (365 µm OD x 50 µm ID) with CE-MS coupling using a liquid sheath-flow interface and isopropanol:methanol (2:1) as the sheath liquid. An aqueous buffer comprising 30 mM morpholine (adjusted to pH9 with formic acid) was used for experiments in the negative-ion mode. Alternatively, mass spectra were acquired using a SQD2 (Waters, Milford, MA). Here, the spectra were collected in the negative ion mode and no separations were attempted. The buffer used was a mixture of 1:1 acetonitrile I water with 0.31 mg/mL of ammonium bicarbonate.

Results verifying the production of each compound are shown in **Table 4** below, where observed mlz ions from MS analysis correspond accurately to the calculated masses.

### Bacterial strains and growth conditions

A mutant of *N. gonorrhoeae* strain F62 [26] that lacked expression of lipooligosaccharide glycosyltransferase D *(lgtD),* called F62 ΔIgtD [27], was provided by Dr. Daniel C. Stein (University of Maryland). LgtD adds a GalNAc residue to the terminal Gal of the Hepl lacto-N-neotetraose species [28]. Therefore, any extension of the Hepl of *N. gonorrhoeae* F62 ΔIgtD is limited to the addition of a nonulosonic acid residue that is transferred from the CMP-nonulosonate added to growth media.

Generally, bacteria (F62 ΔIgtD) grown overnight on chocolate agar plates were suspended in gonococcal liquid media supplemented with IsoVitaleX [29] that contained specified concentrations of the CMP-nonulosonate. Bacteria were then incubated at 37°C for the period specified in each experiment.

### Antibodies

Goat anti-human fH was used in flow cytometry assays to detect human fH binding to bacteria. mAb 3F11 (mouse IgM; provided by Dr. Michael A. Apicella, University of Iowa) binds to the unsialylated Hepl lacto-N-neotetraose structure; sialylation of LOS results in decreased binding of mAb 3F11 [30]. FITC conjugated anti-mouse IgM and anti-goat IgG were from Sigma.

### Flow cytometry or FACS assays

Flow cytometry for fH and mAb 3F11 binding were conducted as described in the art [31] and in U.S. Patent Application Serial No. 14/627,396.

### Animal model experiments

The mouse model experiments and statistical analysis were conducted as described in the art [32] and in U.S. Patent Application Serial No. 14/627,396.

### Serum bactericidal assay

Serum bactericidal assays were performed as follows, similar to methods outlined in [32,33] and in U.S. Patent Application Serial No. 14/627,396. Bacteria were harvested from an overnight culture on chocolate agar plates and ~10⁵ CFU of Ng were grown in liquid media containing the concentrations of CMP-NulO as specified for each experiment. Bacteria were diluted in Morse A and ∼2000 CFU of Ng F62 ΔIgtD were incubated with NHS (concentration specified for each experiment). The final reaction volumes were maintained at 150 µL. Aliquots of 25 µL of reaction mixtures were plated onto chocolate agar in duplicate at the beginning of the assay (t₀) and again after incubation at 37°C for 30 minutes (t₃₀). Survival was calculated as the number of viable colonies at t₃₀ relative to t₀.

Substitution of *Neisseria gonorrhoeae* lacto-N-neotetraose (LNnT) lipooligosaccharide (LOS) with Neu5Ac results in the ability of the bacterium to evade complement-mediated killing. Prior studies have shown that the addition of Neu5Ac to LNnT LOS decreases binding of specific IgG and enhances binding of factor H (fH), an inhibitor of the alternative pathway of complement. Previously, we have shown that several CMP-activated nonulosonate (NulO) analogs, such as CMP-Neu5Gc, CMP-Neu5Gc8Me, CMP-Neu5Ac9Ac, CMP-Neu5Ac9Az and CMP-Leg5Ac7Ac can serve as substrates for gonococcal LOS sialyltransferase (Lst) (U.S. Patent Application Serial No. 14/627,396). From this collection of CMP-NulOs tested, only CMP-Neu5Gc was able to simulate the high-level serum resistance reported for CMP-Neu5Ac, as well as a high level of fH binding to bacteria. Importantly, Neu5Gc differs from Neu5Ac at carbon 5, where it contains an N-glycolyl moiety rather than an N-acetyl one. The remainder of nonulosonates from this collection differ from Neu5Ac/Neu5Gc at either carbon 8, at carbon 9 or at carbons 7 and 9. We found that these CMP-NulO analogs, with changes to carbon 8, carbon 9 or carbons 7 and 9 of the NulO, did not enhance factor H binding, nor did they afford *N. gonorrhoeae* cells a high level of serum resistance. So, it appeared carbons 7, 8 and 9 within the exocyclic moiety of nonulosonate sugars played a critical role in the avoidance of serum mediated killing by *N. gonorrhoeae,* as evidenced by enhanced serum-mediated killing with CMP-Neu5Gc8Me, CMP-Neu5Ac9Ac, CMP-Neu5Ac9Az and CMP-Leg5Ac7Ac that was not observed with either CMP-Neu5Ac or CMP-Neu5Gc only feeding controls. So, in contrast to the other carbon 7, 8 and 9 variations, N-glycolyl substitution at the carbon 5-position did not have any negative impact on fH binding or serum resistance. We therefore proposed the use of CMP-nonulosonate analogs, with variations at carbons 7, 8 and/or 9, as a novel therapeutic/preventative strategy against the global threat of multi-drug resistant gonorrhea.

Surprisingly, we now report a different CMP-NulO with changes to the carbon 5 position of the NulO that can be utilized by *N. gonorrhoeae* Lst, will affect fH binding of bacteria, and result in serum sensitivity. In addition, this CMP-NulO analog was also found to have some efficacy against the antibiotic resistant 'superbug' H041 in the BALB/c vaginal colonization model. This CMP-NulO is CMP-3-deoxy-D-*glycero*-D-*galacto*-nonulosonic acid or CMP-deaminated neuraminic acid, also known as **CMP-KDN.** Like Neu5Gc, KDN differs from Neu5Ac at carbon 5, but unlike Neu5Ac or Neu5Gc with N-acetyl or N-glycolyl groups at carbon 5, respectively, KDN has just a hydroxyl group at carbon 5. An experimental summary for the testing of **CMP-KDN** is described below.

To determine if gonococcal LOS sialyltransferase (Lst) can utilize **CMP-KDN,** *N*. *gonorrhoeae* F62 ΔIgtD was grown in media alone (see 'unsialylated' in **Figure 1A**), or media containing 20 µg/mL of either CMP-Neu5Ac or **CMP-KDN** (**Figure 1A**), and bacteria were screened for binding of mAb 3F11 by flow cytometry. The mAb 3F11 binds to the terminal lactosamine residue of lacto-*N*-neotetraose (LNnT); any extension beyond the terminal Gal (in this instance, with a NulO) would abrogate 3F11 binding. As seen in **Figure 1A****,** growth in media containing **CMP-KDN** or CMP-Neu5Ac decreased binding of mAb 3F11 similarly, indicating that **CMP-KDN** served as a substrate for gonococcal Lst in the context of live bacteria and that KDN was incorporated onto LNnT. In addition, the ability of LNnT incorporated KDN to influence fH binding was examined **(****Figure 1B****).** Here, fH binding to *N. gonorrhoeae* F62 ΔIgtD grown in the presence of 20 µg/mL of either CMP-Neu5Ac or **CMP-KDN** was examined by flow cytometry. Maximal fH binding was seen with Neu5Ac, whereas fH binding with KDN appeared to be half of that observed for Neu5Ac. This is in contrast to testing results with Leg5Ac7Ac, Neu5Ac9Az or Neu5Gc8Me nonulosonates, where they were found to not enhance fH binding above levels seen with an unsialylated F62 ΔlgtD control (U.S. Patent Application Serial No. 14/627,396). So, KDN incorporation only modestly affects fH binding, and is further support for the incorporation of KDN within LNnT LOS. Based on these findings, KDN incorporation within *N. gonorrhoeae* LNnT LOS should not result in high serum sensitivity as that which is observed for Leg5Ac7Ac LNnT LOS incorporation, for example.

Addition of a terminal Neu5Ac residue to the LNnT LOS of *N. gonorrhoeae* that occurs *in vivo* or following the addition of CMP-Neu5Ac to growth media results in resistance to complement-dependent killing [34]. We next determined the effects of LNnT incorporation of KDN on the ability of *N. gonorrhoeae* F62 ΔIgtD to resist complement-dependent killing by normal human sera at concentrations of 3.3% or 10%. Bacteria were grown either in media alone, or media supplemented with 20 µg/mL of CMP-Neu5Ac or **CMP-KDN.** In addition, CMP-NulO competition experiments were also performed with CMP-Neu5Ac, CMP-Leg5Ac7Ac and **CMP-KDN** at CMP-NulO concentrations of either 20 µg/mL or 2 µg/mL as indicated, where the second CMP-NulO was added 15 minutes after the first (**Table 1** below). To note, these CMP-NulO competition experiments are a method to examine the ability of select CMP-NulOs to counter the enhanced serum resistance due to CMP-Neu5Ac addition, providing information on their therapeutic potential (as any potential therapeutic will be required to compete with CMP-Neu5Ac *in vivo).* As shown in **Table 1,** CMP-Leg5Ac7Ac blocked serum resistance mediated by CMP-Neu5Ac at both 3.3% and 10% serum concentrations irrespective of the order of addition (ie CMP-Neu5Ac or CMP-Leg5Ac7Ac first). When **CMP-KDN** was examined alone, serum sensitivity was observed at 10% serum concentration, but complete serum resistance was observed at 3.3% serum concentration. In addition, **CMP-KDN** could only counter CMP-Neu5Ac induced serum resistance if it was provided before CMP-Neu5Ac, and again serum sensitivity was only observed with 10% serum concentrations. These results further suggest that the therapeutic potential of **CMP-KDN** should be lower than CMP-Leg5Ac7Ac.

Regardless of the modest results obtained for **CMP-KDN** above, we still decided to pursue experiments evaluating the *in vivo* efficacy of this analog, somewhat due to the different phenotypes observed for KDN versus Neu5Gc, both NulOs with variation at carbon 5. The efficacy of **CMP-KDN** against the *N. gonorrhoeae* antibiotic resistant 'superbug' H041 was tested in the BALB/c mouse vaginal colonization model [35,32] **(****Figure 2****).**

Four groups of Premarin treated BALB/c mice (10 mice per group) were infected as follows: i) H041 → saline untreated control ('control'), ii) H041 → CMP-Leg5Ac7Ac (10 µg intravaginally daily), iii) H041 → CMP-KDN (10 µg intravaginally daily), and iv) H041 → CMP-Neu5Ac9Az (10 µg intravaginally daily). Treatment with all of these CMP-NulOs significantly attenuated *N. gonorrhoeae* H041 infection. Considering the 'poor' fH binding and bactericidal results obtained with CMP-KDN relative to CMP-Leg5Ac7Ac it is surprising that **CMP-KDN** is just as efficacious in an animal model of colonization.

In efforts to test other **CMP-KDN** analogs, we chose to study **CMP-KDN7N₃,** differing from **CMP-KDN** only at the C7 position of the NulO. Similar to studies with **CMP-KDN,** we found **CMP-KDN7N₃** could be utilized by gonococcal LOS sialyltransferase (Lst) (**Figure 3**) using mAb 3F11 binding studies. In addition, we determined that addition of **CMP-KDN7N₃** to growth media resulted in serum sensitivity of *N. gonorrhoeae* F62 ΔIgtD (albeit only at 10% serum concentrations) (**Table 5**), similar to results obtained with **CMP-KDN** (**Table 1**).

**Table 1. Effect of CMP-KDN on complement killing of N. gonorrhoeae F62 ΔIgtD. CMP-NulO concentrations are shown in parentheses (µg/mL).**

| CMP-NulO added | % survival in | |
|---|---|---|
| | 3.3% serum | 10% serum |
| None | 2 | 3 |
| CMP-Neu5Ac (20) alone | Not done | 115 |
| CMP-KDN (20) alone | 110 | 11 |
| CMP-Neu5Ac (20) →^{A} CMP-KDN (20) | 107 | 108 |
| CMP-KDN (20) →^{A} CMP-Neu5Ac (20) | 107 | 11 |
| CMP-KDN (20) →^{A} CMP-Neu5Ac (2) | 107 | 10 |
| CMP-Neu5Ac (20) →^{A} CMP-Leg5Ac7Ac (20) | 9 | 5 |
| CMP-Leg5Ac7Ac (20) →^{A} CMP-Neu5Ac (20) | 7 | 3 |
| CMP-Leg5Ac7Ac (20) →^{A} CMP-Neu5Ac (2) | 6 | 6 |

| | | |
|---|---|---|
| ^{A} indicates 15 minutes interval before addition of next CMP-NulO. | | |

**Table 2. NMR chemical shifts δ (ppm) for CMP-3-deoxy-D-glycero-D-galacto-nonulosonic acid (CMP-KDN).**

| H3ax | H3ax | | |
|---|---|---|---|
| H3eq | 2.44 | C3 | 42.1 |
| H4 | 4.03 | C4 | 69.7 |
| H5 | 3.60 | C5 | 71.1 |
| H6 | 4.09 | C6 | 74.1 |
| H7 | 3.75 | C7 | 69.7 |
| H8 | 3.94 | C8 | 70.9 |
| H9 | 3.67; 3.92 | C9 | 64.3 |

**Table 3. NMR chemical shifts δ (ppm) for CMP-3,7-dideoxy-7-azido-D-glycero-D-galacto-nonulosonic acid (CMP-KDN7N₃).**

| | | | |
|---|---|---|---|
| H3ax | 1.67 | | |
| H3eq | 2.48 | C3 | 42.0 |
| H4 | 4.03 | C4 | 69.7 |
| H5 | 3.61 | C5 | 72.2 |
| H6 | 4.20 | C6 | 73.8 |
| H7 | 3.82 | C7 | 62.3 |
| H8 | 4.06 | C8 | 69.7 |
| H9 | 3.77; 3.94 | C9 | 64.1 |

**Table 4. MS data for CMP-3-deoxy-D-glycero-D-galacto-nonulosonic acid (CMP-KDN) and CMP-3,7-dideoxy-7-azido-D-glycero-D-galacto-nonulosonic acid (CMP-KDN7N₃).**

| Compound | Observed m/z | Calculated mass | Formula (M) | Comments |
|---|---|---|---|---|
| **CMP-KDN** | 572.4 | 573.4 | C₁₈H₂₈O₁₆N₃P | [M-H]⁻ |
| **CMP-KDN7N₃** | 597.1 | 598.4 | C₁₈H₂₇O₁₅N₆P | [M-H]⁻ |

**Table 5. Effect of CMP-KDN7N₃ on complement killing of N. gonorrhoeae F62 ΔIgtD. CMP-NulO concentrations are shown in parentheses (µg/mL).**

| CMP-NulO added | % survival in | |
|---|---|---|
| | 3.3% serum | 10% serum |
| None | 17.47 | 3.40 |
| CMP-Neu5Ac (20) | 133.03 | 129.02 |
| **CMP-KDN7N₃** (20) | 125.60 | 33.92 |
| **CMP-KDN7N₃** (100) | 123.67 | 22.52 |

### REFERENCES

1. Chen, X., and Varki, A. (2010) ACS Chem. Biol. 5(2), 163-176.
2. Varki, A., and Gagneux, P. (2012) Ann. N Y Acad. Sci. 1253, 16-36.
3. Fearon, D. T. (1978) Proc. Natl. Acad. Sci. USA 75(4), 1971-1975.
4. Kajander, T., Lehtinen, M. J., Hyvarinen, S., Bhattacharjee, A., Leung, E., Isenman, D. E., Meri, S., Goldman, A., and Jokiranta, T. S. (2011) Proc. Natl. Acad. Sci. U S A 108(7), 2897-2902.
5. Seven, E., Hood, D. W., and Thomas, G. H. (2007) Microbiology 153(Pt 9), 2817-2822.
6. Elkins, C., Carbonetti, N. H., Varela, V. A., Stirewalt, D., Klapper, D. G., and Sparling, P. F. (1992) Mol Microbiol 6(18), 2617-2628.
7. Ram, S., Sharma, A. K., Simpson, S. D., Gulati, S., McQuillen, D. P., Pangburn, M. K., and Rice, P. A. (1998) J. Exp. Med. 187(5), 743-752.
8. Wu, H., and Jerse, A. E. (2002) Sialylation of gonococcal LOS occurs during experimental murine gonococcal genital tract infection. In: Caugant, D. A., and Wedege, E. (eds). 13th International Pathogenic Neisseria Conference, Oslo, Norway.
9. Nakata, D., Munster, A-K., Gerardy-Schahn, R., Aoki, N., Matsuda, T., and Kitajima, K. (2001) Glycobiology 11: 685-692.
10. Inoue S. and Kitajima, K. (2006) Glycoconjugate Journal 23: 277-290.
11. Terada, T., Kitazume, S., Kitajima, K., Inoue, S., Ito, F., Troy, F.A., and Inoue, Y. (1993) J. Biol. Chem. 268: 2640-2648.
12. Terada, T., Kitajima, K., Inoue, S., Koppert, K., Brossmer, R., and Inoue, Y. (1996) Eur. J. Biochem. 236: 852-855.
13. Hao, J., Vann, W. F., Hinderlich, S., and Sundaramoorthy, M. (2006) Biochem. J. 397: 195-201.
14. Lawrence, S.M., Huddleston, K.A., Tomiya, N., Nguyen, N., Lee, Y.C., Vann, W.F., Coleman, T.A., and Betenbaugh, M.J. (2001) Glycoconjugate Journal 18: 205-213.
15. Lawrence, S.M., Huddleston, K.A., Pitts, L.R., Nguyen, N., Lee, Y.C., Vann, W.F., Coleman, T.A., and Betenbaugh, M.J. (2000) J. Biol. Chem. 275: 17869-17877.
16. Inoue, S., Lin, S-L., Chang, T., Wu, S-H., Yao, C-W., Chu, T-Y., Troy II, F.A., and Inoue, Y. (1998) J. Biol. Chem. 273: 27199-27204.
17. Inoue, S., Kitajima, K., and Inoue, Y. (1996) J. Biol. Chem. 271: 24341-24344.
18. Go, S., Sato, C., Yin, J., Kannagi, R., and Kitajima, K. (2007) Biochem. Biophys. Res. Commun. 357: 537-542.
19. Yabu, M., Korekane, H., Hatano, K., Kaneda, Y., Nonomura, N., Sato, C., Kitajima, K., and Miyamoto, Y. (2013) Glycobiology 23: 634-642.
20. Angata, T., Matsuda, T., and Kitajima, K. (1998) Glycobiology 8: 277-284.
21. Khedri, Z., Muthana, M.M., Li, Y., Muthana, S.M., Yu, H., Cao, H., and Chen, X. (2012) Chem. Commun. 48: 3357-3359.
22. Chan, T-H. and Li C-J. (1992) J. Chem. Soc., Chem. Commun. 747-748.
23. Crich, D. and Navuluri, C. (2011) Org. Lett. 13: 6288-6291.
24. Li, Y.; Yu, H.; Cao, H.; Lau, K.; Muthana, S.; Tiwari, V.K.; Son, B.; Chen, X. (2008) Appl. Microbiol. Biotechnol. 79(6), 963-970.
25. Guerry, P.; Ewing, C.P.; Hickey, T.E.; Prendergast, M.M.; Moran, A.P. (2000) Infect. Immun. 68(12), 6656-6662.
26. Schneider, H., Griffiss, J. M., Williams, G. D., and Pier, G. B. (1982) J. Gen. Microbiol. 128(Pt 1), 13-22.
27. Song, W., Ma, L., Chen, R., and Stein, D. C. (2000) J. Exp. Med. 191(6), 949-960.
28. Yang, Q. L., and Gotschlich, E. C. (1996) J. Exp. Med. 183(1), 323-327.
29. McQuillen, D. P., Gulati, S., and Rice, P. A. (1994) Methods Enzymol. 236, 137-147.
30. Yamasaki, R., Nasholds, W., Schneider, H., and Apicella, M. A. (1991) Mol. Immunol. 28(11), 1233-1242.
31. Lewis LA, Vu DM, Vasudhev S, Shaughnessy J, Granoff DM, Ram S. MBio. 2013 Oct 15;4(5):e00339-13. doi: 10.1128/mBio.00339-13.
32. Gulati S, Zheng B, Reed GW, Su X, Cox AD, St Michael F, Stupak J, Lewis LA, Ram S, Rice PA. PLoS Pathog. 2013;9(8):e1003559. doi: 10.1371/journal.ppat.1003559. Epub 2013 Aug 29.
33. McQuillen DP, Gulati S, Rice PA. Methods Enzymol. 1994;236:137-47.
34. Smith, H., Cole, J.A., and Parsons, N.J. (1992) FEMS Micobiol. Lett. 79(1-3), 287-292.
35. Jerse A.E., Wu, H., Packiam, M., Vonck, R.A., Begum, A.A., and Garvin, L.E. Front Microbial. 2011; 2:107.
36. Campanero-Rhodes, M.A., Solis, D., Carrera, E., de la Cruz, M.J., and Diaz-Maurino, T. (1999) Glycobiology 9: 527-532.
37. Go, S., Sato, C., Furuhata, K., and Kitajima, K. (2006) Glycocon. J. 23: 411-421.
38. Haselhorst, T., Munster-Kuhnel, A.K., Stolz, A., Oschlies, M., Tiralongo, J., Kitajima, K., Gerardy-Schahn, R., and von Itzstein, M. (2005) Biochem. Biophys. Res. Commun. 327: 565-570.
39. Hayakawa, T. and Varki A. (2012) Chapter 8 Human-specific changes in sialic acid biology. Post-Genome Biology of Primates, primatology Monographs, H. Hirai et al. (eds.) Springer.
40. Khedri, Z., Li, Y., Muthana, S., Muthana, M.M., Hsiao, C-W., Yu, H., and Chen, X. (2014) Carbohydrate Research 389: 100-111.
41. Padler-Karavani, V., Song, X., Yu, H., Hurtado-Ziola, N., Huang, S., Muthana, S., Chokhawala, H.A., Cheng, J., Verhagen, A., Langereis, M.A., Kleene, R., Schachner, M., de Groot, R.J., Lasanajak, Y., Matsuda, H., Schwab, R., Chen, X., Smith, D.F., Cummings, R.D., and Varki, A. (2012) J. Biol. Chem. 287: 22593-22608.
42. Schaper, W., Bentrop, J., Ustinova, J., Blume, L., Kats, E., Tiralongo, J., Weinhold, B., Bastmeyer, M., and Munster-Kuhnel, A-K. (2012) J. Biol. Chem. 287: 13239-13248.
43. Song, X., Yu, H., Chen, X., Lasanajak, Y., Tappert, M.M., Air, G.M., Tiwari, V.K., Cao, H., Chokhawala, H.A., Zheng, H., Cummings, R.D., and Smith, D.F. (2011) J. Biol. Chem. 286: 31610-31622.
44. Yu, S., Kojima, N., Hakomori, S-I., Kudo, S., Inoue, S., and Inoue Y. (2002) Proc. Nat. Acad. Sci. USA 99: 2854-2859.
45. Zanetta, J-P., Pons, A., Iwersen, M., Mariller, C., Leroy, Y., Timmerman, P., and Schauer, R. (2001) Glycobiology 11: 663-676.
46. Gulati et al. PLOS Pathogens doi:10.1371/journal.ppat.1005290, December 2, 2015.

## Claims

1. A compound of general formula IIA below or a pharmaceutical composition comprising said compound, or a pharmaceutically acceptable salt thereof, or a solvate or hydrate thereof, or a stereoisomer thereof, for use in the treatment or prevention of a *Neisseria gonorrhoeae* infection in a subject 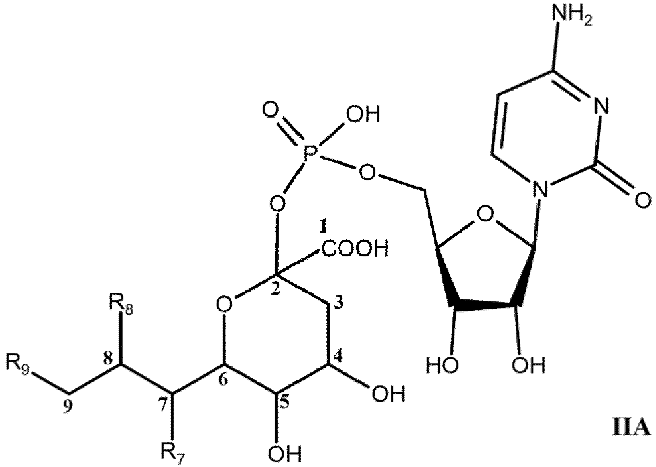 wherein:
R₇ to R₉ are each independently selected from the group consisting of: H; OR wherein R is H or a C₁ to C₆ linear, branched, saturated or unsaturated alkyl or cycloalkyl; XCYR wherein X and Y are each independently O or S and R is a C₁ to C₆ linear, branched, saturated or unsaturated alkyl or cycloalkyl or R is a substituted or unsubstituted phenyl or alkyl phenyl; NR'R" wherein R' and R" are each independently H, a C₁ to C₆ linear, branched, saturated or unsaturated alkyl or cycloalkyl, or R' and R" together with N form a 5- or 6-member ring, optionally the ring is substituted with a C₁ to C₃ alkyl; NH-acetyl; NH-thio-acetyl; NH-azido-acetyl; NH-(D-alanyl); NH-(N-acetyl-D-alanyl); N₃; O-Sia; O-Glc; benzamido [NHCOPh]; NH-Gly; NH-Succ; hexanoylamido [NHCO(CH₂)₄CH₃]; O-lactyl; O-phosphate; O-sulfate; and a halogen atom which is F, Cl, Br or I.

2. The compound or pharmaceutical composition for use according to claim 1, wherein:
R₇ is OH, NH₂, O-acetyl, O-methyl, NH-acetyl, NH-azido-acetyl, NH-(D-alanyl), NH-(N-acetyl-D-alanyl), F, H, or N₃;
R₈ is OH, NH₂, N₃, O-acetyl, O-methyl, O-sulfate, O-Sia, or O-Glc; and
R₉ is OH, O-acetyl, N₃, NH₂, NH-acetyl, NH-thio-acetyl, benzamido [NHCOPh], NH-Gly, NH-Succ, hexanoylamido [NHCO(CH₂)₄CH₃], O-methyl, O-lactyl, O-phosphate, O-sulfate, O-Sia, F or H.

3. The compound or pharmaceutical composition for use according to claim 1, wherein the compound is compound V below

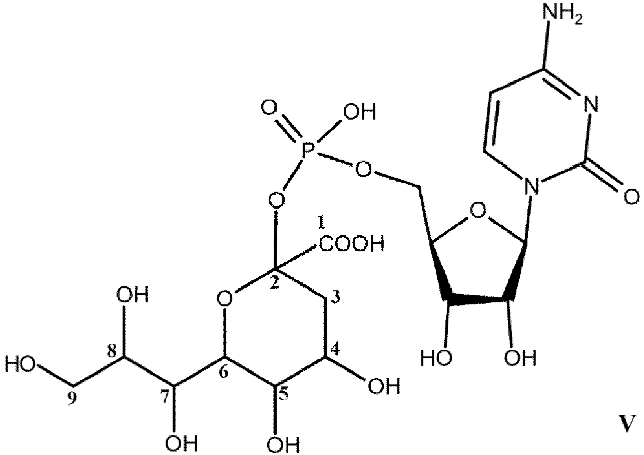

4. The compound or pharmaceutical composition for use according to claim 1, wherein the compound is cytidine 5'-monophospho-3-deoxy-D-glycero-D-galacto-nonulosonic acid **(CMP-KDN)** below

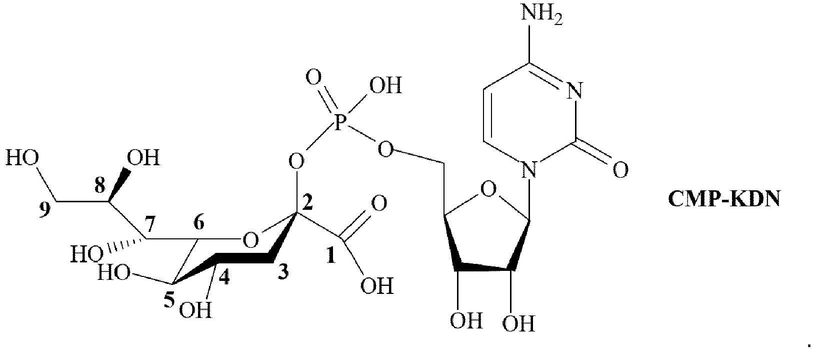

5. The compound or pharmaceutical composition for use according to claim 1, wherein the compound is cytidine 5'-monophospho-3,7-dideoxy-7-azido-D-*glycero*-D-*galacto-*nonulosonic acid **(CMP-KDN7N₃)** below

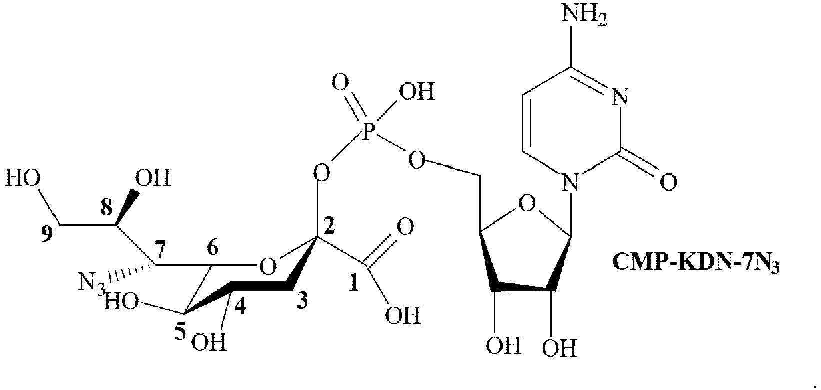

6. The compound or pharmaceutical composition for use according to claim 1, wherein the compound is:
cytidine 5'-monophospho-3-deoxy-9-O-acetyl-D-glycero-D-galacto-nonulosonic acid (CMP-KDN9OAc) (R₇ = OH, R₈ = OH, R₉ = O-acetyl);
cytidine 5'-monophospho-3-deoxy-8-O-acetyl-D-*glycero*-D-*galacto*-nonulosonic acid (CMP-KDN8OAc) (R₇ = OH, R₈ = O-acetyl, R₉= OH);
cytidine 5'-monophospho-3-deoxy-7-O-acetyl-D-*glycero*-D-*galacto*-nonulosonic acid (CMP-KDN7OAc) (R₇ = O-acetyl, R₈ = OH, R₉= OH);
cytidine 5'-monophospho-3-deoxy-8,9-di-O-acetyl-D-*glycero*-D-*galacto*-nonulosonic acid (CMP-KDN8,9diOAc) (R₇ = OH, R₈ = O-acetyl, R₉ = O-acetyl);
cytidine 5'-monophospho-3-deoxy-9-O-methyl-D-*glycero*-D-*galacto*-nonulosonic acid (CMP-KDN9OMe) (R₇ = OH, R₈ = OH, R₉= O-methyl);
cytidine 5'-monophospho-3-deoxy-8-O-methyl-D-*glycero*-D-*galacto*-nonulosonic acid (CMP-KDN8OMe) (R₇ = OH, R₈ = O-methyl, R₉= OH);
cytidine 5'-monophospho-3-deoxy-7-O-methyl-D-*glycero*-D-*galacto*-nonulosonic acid (CMP-KDN7OMe) (R₇ = O-methyl, R₈ = OH, R₉= OH);
cytidine 5'-monophospho-3-deoxy-8,9-di-O-methyl-D-*glycero*-D-*galacto*-nonulosonic acid (CMP-KDN8,9diOMe) (R₇ = OH, R₈ = O-methyl, R₉= O-methyl);
cytidine 5'-monophospho-3,9-dideoxy-D-*glycero*-D-*galacto*-nonulosonic acid (CMP-9-deoxy-KDN) (R₇ = OH, R₈ = OH, R₉= H);
cytidine 5'-monophospho-3,7-dideoxy-D-*glycero*-D-*galacto*-nonulosonic acid (CMP-7-deoxy-KDN) (R₇ = H, R₈ = OH, R₉= OH);
cytidine 5'-monophospho-3,9-dideoxy-9-azido-D-*glycero*-D-*galacto*-nonulosonic acid (CMP-KDN9Az) (R₇ = OH, R₈ = OH, R₉= N₃);
cytidine 5'-monophospho-3,9-dideoxy-9-fluoro-D-*glycero*-D-*alacto*-nonulosonic acid (CMP-KDN9F) (R₇ = OH, R₈ = OH, R₉= F); or
cytidine 5'-monophospho-3,7-dideoxy-7-fluoro-D-*glycero*-D-*galacto*-nonulosonic acid (CMP-KDN7F) (R₇ = F, R₈ = OH, R₉= OH).

7. The pharmaceutical composition for use according to any one of claims 1 to 6, wherein the pharmaceutical composition comprises the compound and a pharmaceutically acceptable carrier.

8. The pharmaceutical composition for use according to any one of claims 1 to 6, wherein the pharmaceutical composition comprises the compound and another therapeutic compound.

9. The pharmaceutical composition for use according to claim 8, wherein the another therapeutic compound is selected from the group consisting of: other compounds used in the treatment or prevention of *Neisseria gonorrhoeae* infection, compounds used in the treatment or prevention of sexually transmitted diseases including *Chlamydia trachomatis* infection and HIV, and antibacterial peptides.

10. The compound or pharmaceutical composition for use according to any one of claims 1 to 9, wherein the subject is a mammal.

11. The compound or pharmaceutical composition for use according to claim 10, wherein the subject is a human.

12. A device coated or filled with a compound as defined in any one of claims 1 to 6 or a pharmaceutical composition as defined in any one of claims 1 to 11 for use according to claim 1.

## Patentansprüche

1. Verbindung der allgemeinen Formel **IIA** unten oder eine pharmazeutische Zusammensetzung, umfassend die Verbindung oder ein pharmazeutisch akzeptables Salz davon, oder ein Solvat oder Hydrat davon, oder ein Stereoisomer davon, zur Verwendung bei der Behandlung oder Prävention einer *Neisseria gonorrhoeae-*Infektion in einem Individuum 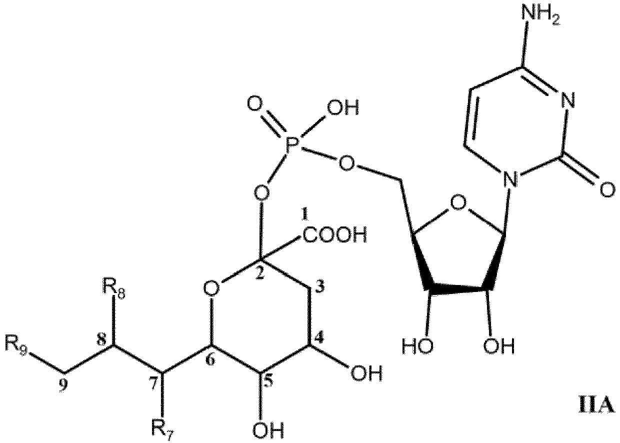 wobei:
R₇ bis R₉ jeweils unabhängig ausgewählt sind aus der Gruppe bestehend aus: H; OR, wobei R H oder ein lineares, verzweigtes, gesättigtes oder ungesättigtes C₁- bis C₆-Alkyl oder -Cycloalkyl ist; XCYR, wobei X und Y jeweils unabhängig O oder S sind und R ein lineares, verzweigtes, gesättigtes oder ungesättigtes C₁- bis C₆-Alkyl oder -Cycloalkyl ist oder R ein substituiertes oder unsubstituiertes Phenyl oder Alkylphenyl ist; NR'R", wobei R' und R" jeweils unabhängig H, ein lineares, verzweigtes, gesättigtes oder ungesättigtes C₁- bis C₆-Alkyl oder -Cycloalkyl sind, oder R' und R" zusammen mit N einen 5- oder 6-gliedrigen Ring bilden, wobei optional der Ring mit einem C₁- bis C₃-Alkyl substituiert ist; NH-Acetyl; NH-Thioacetyl; NH-Azidoacetyl; NH-(D-Alanyl); NH-(N-Acetyl-D-alanyl); N₃; O-Sia; O-Glc; Benzamido [NHCOPh]; NH-Gly; NH-Succ; Hexanoylamido [NHCO(CH₂)₄CH₃]; O-Lactyl; O-Phosphat; O-Sulfat; und einem Halogenatom, welches F, Cl, Br oder I ist.

2. Verbindung oder pharmazeutische Zusammensetzung zur Verwendung nach Anspruch 1, wobei:
R₇ OH, NH₂, O-Acetyl, O-Methyl, NH-Acetyl, NH-Azido-acetyl, NH-(D-Alanyl), NH-(N-Acetyl-D-alanyl), F, H oder N₃ ist;
R₈ OH, NH₂, N₃, O-Acetyl, O-Methyl, O-Sulfat, O-Sia oder O-Glc ist; und
R₉ OH, O-Acetyl, N₃, NH₂, NH-Acetyl, NH-Thioacetyl, Benzamido [NHCOPh], NH-Gly, NH-Succ, Hexanoylamido [NHCO(CH₂)₄CH₃], O-Methyl, O-Lactyl, O-Phosphat, O-Sulfat, O-Sia, F oder H ist.

3. Verbindung oder pharmazeutische Zusammensetzung zur Verwendung nach Anspruch 1, wobei die Verbindung Verbindung V unten ist

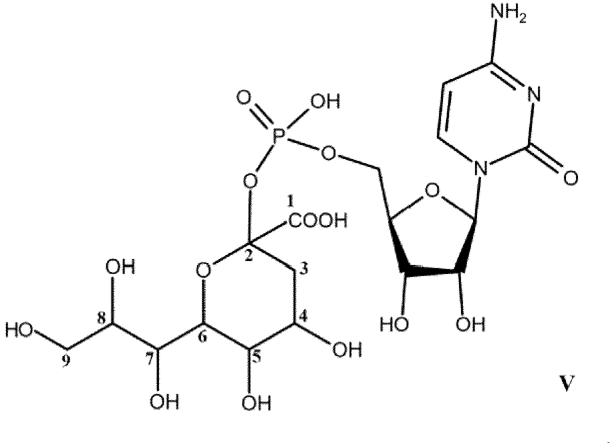

4. Verbindung oder pharmazeutische Zusammensetzung zur Verwendung nach Anspruch 1, wobei die Verbindung Cytidin-5'-monophospho-3-desoxy-D-*glycero*-D-*galacto*-nonulosonsäure **(CMP-KDN)** wie folgt ist

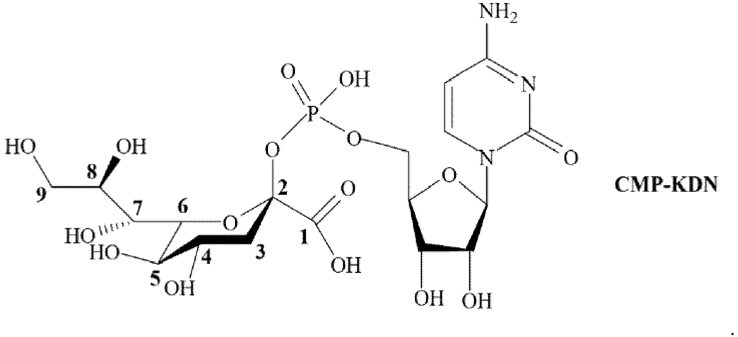

5. Verbindung oder pharmazeutische Zusammensetzung zur Verwendung nach Anspruch 1, wobei die Verbindung Cytidin-5'-monophospho-3,7-dideoxy-7-azido-D-*glycero*-D-*galacto*-nonulosonsäure **(CMP-KDN7N₃)** unten ist

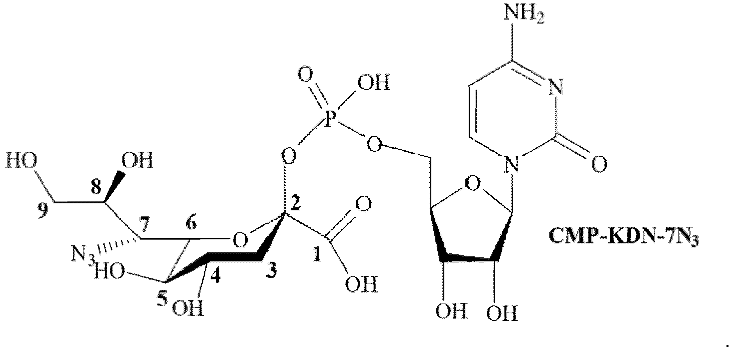

6. Verbindung oder pharmazeutische Zusammensetzung zur Verwendung nach Anspruch 1, wobei die Verbindung:
Cytidin-5'-monophospho-3-desoxy-9-O-acetyl-D-*glycero*-D-*galacto*-nonulosonsäure (CMP-KDN9OAc) (R₇ = OH, R₈ = OH, R₉ = O-Acetyl);
Cytidin-5'-monophospho-3-desoxy-8-O-acetyl-D-*glycero*-D-*galacto*-nonulosonsäure (CMP-KDN8OAc) (R₇ = OH, R₈ = O-Acetyl, R₉ =OH);
Cytidin-5'-monophospho-3-desoxy-7-0-acetyl-D-*glycero*-D-*galacto*-nonulosonsäure (CMP-KDN7OAc) (R₇ = O-Acetyl, R₈ = OH, R₉ =OH);
Cytidin-5'-monophospho-3-desoxy-8,9-di-O-acetyl-D-*glycero*-D-*galacto-*nonulosonsäure (CMP-KDN8,9diOAc) (R₇ = OH, R₈ = O-Acetyl, R₉ = O-Acetyl);
Cytidin-5'-monophospho-3-desoxy-9-O-methyl-D-*glycero*-D-*galacto*-nonulosonsäure (CMP-KDN9OMe) (R₇ = OH, R₈ = OH, R₉ = O-Methyl);
Cytidin-5'-monophospho-3-desoxy-8-O-methyl-D-*glycero*-D-*galacto*-nonulosonsäure (CMP-KDN8OMe) (R₇ = OH, R₈ = O-Methyl, R₉ = OH);
Cytidin-5'-monophospho-3-desoxy-7-O-methyl-D-*glycero*-D-*galacto*-nonulosonsäure (CMP-KDN7OMe) (R₇ = O-Methyl, R₈ = OH, R₉ = OH);
Cytidin-5'-monophospho-3-desoxy-8,9-di-O-methyl-D-*glycero*-D-*galacto-*nonulosonsäure (CMP-KDN8,9diOMe) (R₇ = OH, R₈ = O-Methyl, R₉ = O-Methyl);
Cytidin-5'-monophospho-3,9-didesoxy-D-*glycero*-D-*galacto*-nonulosonsäure (CMP-9-desoxy-KDN) (R₇ = OH, R₈ = OH, R₉ = H);
Cytidin-5'-monophospho-3,7-didesoxy-D-*glycero*-D-*galacto*-nonulosonsäure (CMP-7-desoxy-KDN) (R₇ = H, R₈ = OH, R₉ = OH);
Cytidin-5'-monophospho-3,9-didesoxy-9-azido-D-*glycero*-D-*galacto*-nonulosonsäure (CMP-KDN9Az) (R₇ = OH, R₈ = OH, R₉ = N₃);
Cytidin-5'-monophospho-3,9-didesoxy-9-fluor-D-*glycero*-D-*galacto*-nonulosonsäure (CMP-KDN9F) (R₇ = OH, R₈ = OH, R₉ = F); oder
Cytidin-5'-monophospho-3,7-didesoxy-7-fluor-D-*glycero*-D-*galacto*-nonulosonsäure (CMP-KDN7F) (R₇= F, R₈ = OH, R₉ = OH) ist.

7. Pharmazeutische Zusammensetzung zur Verwendung nach einem der Ansprüche 1 bis 6, wobei die pharmazeutische Zusammensetzung die Verbindung und einen pharmazeutisch akzeptablen Träger umfasst.

8. Pharmazeutische Zusammensetzung zur Verwendung nach einem der Ansprüche 1-6, wobei die pharmazeutische Zusammensetzung die Verbindung und eine andere therapeutische Verbindung umfasst.

9. Pharmazeutische Zusammensetzung zur Verwendung nach Anspruch 8, wobei die andere therapeutische Verbindung ausgewählt ist aus der Gruppe bestehend aus: anderen Verbindungen, welche bei der Behandlung oder Prävention von *Neisseria gonorrhoeae*-Infektion verwendet werden, Verbindungen, welche bei der Behandlung oder Prävention von sexuell übertragbaren Krankheiten, umfassend *Chlamydia trachomatis-*Infektion und HIV, verwendet werden, und antibakteriellen Peptiden.

10. Verbindung oder pharmazeutische Zusammensetzung zur Verwendung nach einem der Ansprüche 1 bis 9, wobei das Individuum ein Säugetier ist.

11. Verbindung oder pharmazeutische Zusammensetzung zur Verwendung nach Anspruch 10, wobei das Individuum ein Mensch ist.

12. Vorrichtung, beschichtet oder gefüllt mit einer Verbindung wie in einem der Ansprüche 1 bis 6 definiert oder einer pharmazeutischen Zusammensetzung wie in einem der Ansprüche 1 bis 11 definiert, zur Verwendung nach Anspruch 1.

## Revendications

1. Composé de formule générale IIA ci-dessous ou composition pharmaceutique comprenant ledit composé, ou sel pharmaceutiquement acceptable de celui-ci, ou solvate ou hydrate de celui-ci, ou stéréoisomère de celui-ci, pour une utilisation dans le traitement ou la prévention d'une infection à *Neisseria gonorrhoeae* chez un sujet 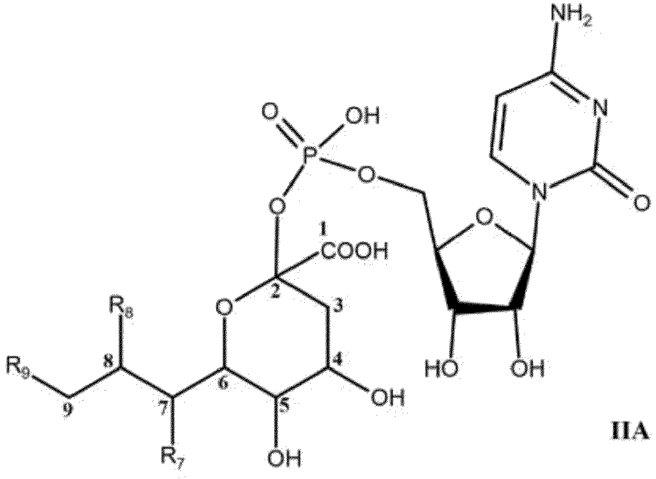 dans laquelle :
R₇ à R₉ sont chacun indépendamment choisis dans le groupe constitué de : H ; OR dans lequel R est H ou un alkyle ou cycloalkyle en C₁ à C₆ linéaire, ramifié, saturé ou insaturé ; XCYR dans lequel X et Y sont chacun indépendamment O ou S et R est un alkyle ou cycloalkyle en C₁ à C₆ linéaire, ramifié, saturé ou insaturé ou R est un phényle ou alkylphényle substitué ou non substitué ; NR'R" dans lequel R' et R" sont chacun indépendamment H, un alkyle ou cycloalkyle en C₁ à C₆ linéaire, ramifié, saturé ou insaturé, ou R' et R" forment conjointement avec N un cycle à 5 ou 6 chaînons, le cycle est éventuellement substitué par un alkyle en C₁ à C₃ ; NH-acétyle ; NH-thio-acétyle ; NH-azido-acétyle ; NH-(D-alanyle) ; NH-(N-acétyl-D-alanyle) ; N₃ ; O-Sia ; O-Glc ; benzamido [NHCOPh] ; NH-Gly ; NH-Succ ; hexanoylamido [NHCO(CH₂)₄CH₃] ; O-lactyle ; O-phosphate ; O-sulfate ; et un atome d'halogène qui est F, CI, Br ou I.

2. Composé ou composition pharmaceutique pour une utilisation selon la revendication 1, dans lequel :
R₇ est OH, NH₂, O-acétyle, O-méthyle, NH-acétyle, NH-azido-acétyle, NH-(D-alanyle), NH-(N-acétyl-D-alanyle), F, H ou N₃ ;
R₈ est OH, NH₂, N₃, O-acétyle, O-méthyle, O-sulfate, O-Sia ou O-Glc ; et
R₉ est OH, O-acétyle, N₃, NH₂, NH-acétyle, NH-thio-acétyle, benzamido [NHCOPh], NH-Gly, NH-Succ, hexanoylamido [NHCO(CH₂)₄CH₃], O-méthyle, O-lactyle, O-phosphate, O-sulfate, O-Sia, Fou H.

3. Composé ou composition pharmaceutique pour une utilisation selon la revendication 1, dans lequel le composé est le composé V ci-dessous

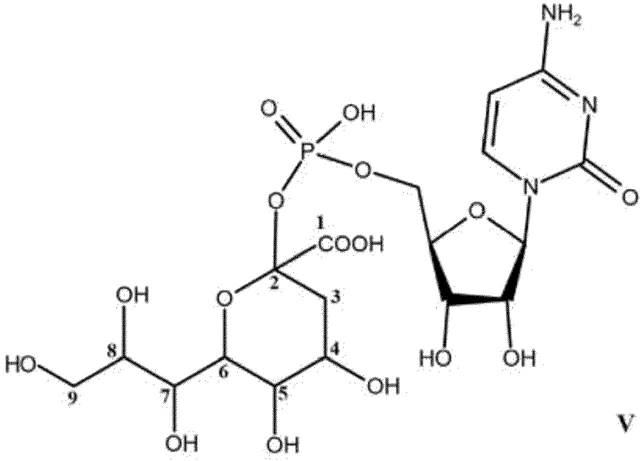

4. Composé ou composition pharmaceutique pour une utilisation selon la revendication 1, dans lequel le composé est l'acide cytidine 5'-monophospho-3-désoxy-D-*glycéro*-D-*galacto*-nonulosonique (CMP-KDN) ci-dessous

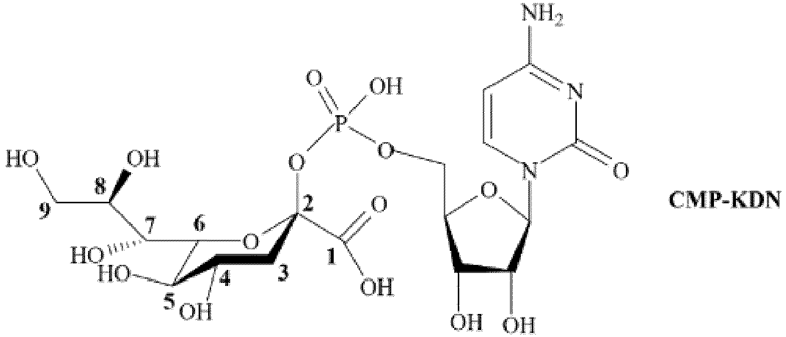

5. Composé ou composition pharmaceutique pour une utilisation selon la revendication 1, dans lequel le composé est l'acide cytidine 5'-monophospho-3,7-didésoxy-7-azido-D-*glycéro*-D-*galacto*-nonulosonique (CMP-KDN7N₃) ci-dessous

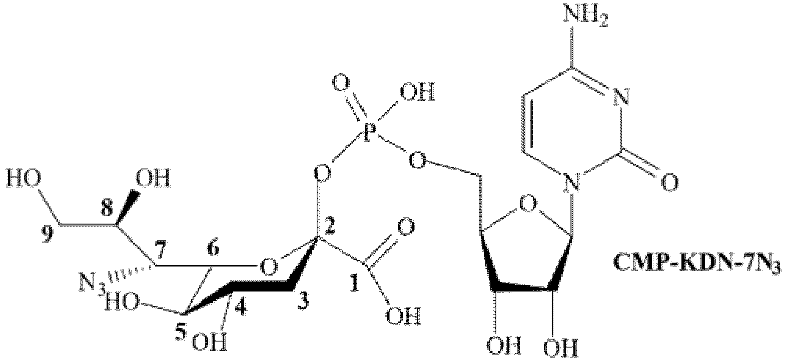

6. Composé ou composition pharmaceutique pour une utilisation selon la revendication 1, dans lequel le composé est :
l'acide cytidine 5'-monophospho-3-désoxy-9-O-acétyl-D-*glycéro*-D-*galacto-*nonulosonique (CMP-KDN9OAc) (R₇ = OH, R₈ = OH, R₉ = O-acétyle) ;
l'acide cytidine 5'-monophospho-3-désoxy-8-O-acétyl-D-*glycéro*-D-*galacto-*nonulosonique (CMP-KDN8OAc) (R₇ = OH, R₈ = O-acétyle, R₉ = OH) ;
l'acide cytidine 5'-monophospho-3-désoxy-7-O-acétyl-D-*glycéro*-D-*galacto-*nonulosonique (CMP-KDN7OAc) (R₇ = O-acétyle, R₈ = OH, R₉ = OH) ;
l'acide cytidine 5'-monophospho-3-désoxy-8,9-di-O-acétyl-D-*glycéro*-D-*galacto*-nonulosonique (CMP-KDN8, 9diOAc) (R₇ = OH, R₈ = O-acétyle, R₉ = O-acétyle) ;
l'acide cytidine 5'-monophospho-3-désoxy-9-O-méthyl-D-*glycéro*-D-*galacto-*nonulosonique (CMP-KDN9OMe) (R₇ = OH, R₈ = OH, R₉ = O-méthyle) ;
l'acide cytidine 5'-monophospho-3-désoxy-8-O-méthyl-D-*glycéro*-D-*galacto-*nonulosonique (CMP-KDN8OMe) (R₇ = OH, R₈ = O-méthyle, R₉ = OH) ;
l'acide cytidine 5'-monophospho-3-désoxy-7-O-méthyl-D-*glycéro*-D-*galacto-*nonulosonique (CMP-KDN7OMe) (R₇ = O-méthyle, R₈ = OH, R₉ = OH) ;
l'acide cytidine 5'-monophospho-3-désoxy-8,9-di-O-méthyl-D-*glycéro*-D-*galacto*-nonulosonique (CMP-KDN8,9diOMe) (R₇ = OH, R₈ = O-méthyle, R₉ = O-méthyle) ;
l'acide cytidine 5'-monophospho-3,9-didésoxy-D-*glycéro*-D-*galacto-*nonulosonique (CMP-9-désoxy-KDN) (R₇ = OH, R₈ = OH, R₉ = H) ;
l'acide cytidine 5'-monophospho-3,7-didésoxy-D-*glycéro*-D-*galacto-*nonulosonique (CMP-7-désoxy-KDN) (R₇ = H, R₈ = OH, R₉ = OH) ;
l'acide cytidine 5'-monophospho-3,9-didésoxy-9-azido-D-*glycéro*-D-*galacto-*nonulosonique (CMP-KDN9Az) (R₇ = OH, R₈ = OH, R₉ = N₃) ;
l'acide cytidine 5'-monophospho-3,9-didésoxy-9-fluoro-D-*glycéro*-D-*galacto-*nonulosonique (CMP-KDN9F) (R₇ = OH, R₈ = OH, R₉ = F) ; ou
l'acide cytidine 5'-monophospho-3,7-didésoxy-7-fluoro-D-*glycéro*-D-*galacto-*nonulosonique (CMP-KDN7F) (R₇ = F, R₈ = OH, R₉ = OH).

7. Composition pharmaceutique pour une utilisation selon l'une quelconque des revendications 1 à 6, dans laquelle la composition pharmaceutique comprend le composé et un support pharmaceutiquement acceptable.

8. Composition pharmaceutique pour une utilisation selon l'une quelconque des revendications 1 à 6, dans laquelle la composition pharmaceutique comprend le composé et un autre composé thérapeutique.

9. Composition pharmaceutique pour une utilisation selon la revendication 8, dans laquelle l'autre composé thérapeutique est choisi dans le groupe constitué par : d'autres composés utilisés dans le traitement ou la prévention d'une infection à *Neisseria gonorrhoeae,* des composés utilisés dans le traitement ou la prévention de maladies sexuellement transmissibles, y compris une infection à *Chlamydia trachomatis* et le VIH, et des peptides antibactériens.

10. Composé ou composition pharmaceutique pour une utilisation selon l'une quelconque des revendications 1 à 9, dans lequel le sujet est un mammifère.

11. Composé ou composition pharmaceutique pour une utilisation selon la revendication 10, dans lequel le sujet est un humain.

12. Dispositif revêtu ou rempli d'un composé tel que défini selon l'une quelconque des revendications 1 à 6 ou d'une composition pharmaceutique telle que définie selon l'une quelconque des revendications 1 à 11 pour une utilisation selon la revendication 1.
